# EUROPEAN PATENT SPECIFICATION

(11) **EP 3 982 960 B1**
(45) Date of publication and mention of the grant of the patent: **28.06.2023**
(21) Application number: 20734283.3
(22) Date of filing: 15.06.2020
(51) Int. Cl.: A61K 31/444, A61P 7/00

(54) **TREATMENTS OF HEREDITARY ANGIOEDEMA**
BEHANDLUNG VON HEREDITÄREM ANGIOÖDEM
TRAITEMENTS DE L'ANGIO-OEDÈME HÉRÉDITAIRE

(30) Priority: 14.06.2019 US 201962861725 P; 15.07.2019 GB 201910116
(43) Date of publication of application: 20.04.2022
(73) Proprietor: Kalvista Pharmaceuticals Limited, Porton Down, Salisbury SP4 0BF Wiltshire (GB)
(72) Inventor: FEENER, Edward Paul, North Reading, Massachusetts 01864 (US); MARSH, Sally Louise, Salisbury, Wiltshire SP4 0BF (GB); MAETZEL, Andreas, Chapel Hill, North Carolina 27516 (US); SMITH, Michael David, Salt Lake City, Utah 84109 (US); YEA, Christopher Martyn, Salisbury, Wiltshire SP4 0BF (GB)
(74) Representative: Carpmaels & Ransford LLP
(86) International application number: PCT/GB2020/051439
(87) International publication number: WO 2020/249977

(56) References cited:
- WO-A1-2019/106361

## Description

The present invention relates to treatments of hereditary angioedema (HAE). In particular, the present invention provides a compound of Formula A for use in on-demand treatments of hereditary angioedema (HAE) by orally administering a plasma kallikrein inhibitor to a patient in need thereof on-demand.

### Background to the invention

Inhibitors of plasma kallikrein have a number of therapeutic applications, particularly in the treatment of hereditary angioedema.

Plasma kallikrein is a trypsin-like serine protease that can liberate kinins from kininogens (see K. D. Bhoola et al., "Kallikrein-Kinin Cascade", Encyclopedia of Respiratory Medicine, p483-493; J. W. Bryant et al., "Human plasma kallikrein-kinin system: physiological and biochemical parameters" Cardiovascular and haematological agents in medicinal chemistry, 7, p234-250, 2009; K. D. Bhoola et al., Pharmacological Rev., 1992, 44, 1; and D. J. Campbell, "Towards understanding the kallikrein-kinin system: insights from the measurement of kinin peptides", Brazilian Journal of Medical and Biological Research 2000, 33, 665-677). It is an essential member of the intrinsic blood coagulation cascade although its role in this cascade does not involve the release of bradykinin or enzymatic cleavage. Plasma prekallikrein is encoded by a single gene and can be synthesized in the liver, as well as other tissues. It is secreted by hepatocytes as an inactive plasma prekallikrein that circulates in plasma as a heterodimer complex bound to high molecular weight kininogen (HK) which is activated to give the active plasma kallikrein. This contact activation system (or contact system) can be activated by negatively charged surfaces that activate Factor XII (FXII) to Factor XIIa (FXlla), by certain proteases e.g. plasmin (Hofman et al Clin Rev Allergy Immunol 2016), which may not require negative surfaces, or by misfolded proteins (Maas et al J Clinical Invest 2008). FXlla mediates conversion of plasma prekallikrein to plasma kallikrein and the subsequent cleavage of high molecular weight kininogen (HK) to generate bradykinin, a potent inflammatory hormone. Kinins are potent mediators of inflammation that act through G protein-coupled receptors and antagonists of kinins (such as bradykinin receptor antagonists) have previously been investigated as potential therapeutic agents for the treatment of a number of disorders (F. Marceau and D. Regoli, Nature Rev., Drug Discovery, 2004, 3, 845-852).

Plasma kallikrein is thought to play a role in a number of inflammatory disorders. The major inhibitor of plasma kallikrein is the serpin C1 esterase inhibitor. Patients who present with a genetic deficiency in C1 esterase inhibitor suffer from hereditary angioedema (HAE) which results in intermittent swelling of face, hands, throat, gastro-intestinal tract and genitals. Blisters formed during acute episodes contain high levels of plasma kallikrein which cleaves high molecular weight kininogen (HK) liberating bradykinin leading to increased vascular permeability. Treatment with a large protein plasma kallikrein inhibitor has been shown to effectively treat HAE by preventing the release of bradykinin which causes increased vascular permeability (A. Lehmann "Ecallantide (DX-88), a plasma kallikrein inhibitor for the treatment of hereditary angioedema and the prevention of blood loss in on-pump cardiothoracic surgery" Expert Opin. Biol. Ther. 8, p1187-99).

Hereditary angioedema is a rare inherited disorder characterised by recurrent acute attacks where fluids accumulate outside of the blood vessels, blocking the normal flow of blood or lymphatic fluid and causing rapid swelling of tissues such as in the hands, feet, limbs, face, intestinal tract, or airway. "Hereditary angioedema" can thus be defined as any disorder characterised by recurrent episodes of bradykinin-mediated angioedema (e.g. severe swelling) caused by an inherited dysfunction/fault/mutation. There are currently three known categories of HAE: (i) HAE type 1, (ii) HAE type 2, and (iii) normal C1 inhibitor HAE (normal C1-Inh HAE). However, the HAE field is developing quickly so it is expected that further types of HAE might be defined in the future.

Without wishing to be bound by theory, it is thought that HAE type 1 is caused by mutations in the *SERPING1* gene that lead to reduced levels of C1 inhibitor in the blood. Without wishing to be bound by theory, it is thought that HAE type 2 is caused by mutations in the *SERPING1* gene that lead to dysfunction of the C1 inhibitor in the blood. Without wishing to be bound by theory, the cause of normal C1-Inh HAE is less well defined and the underlying genetic dysfunction/fault/mutation can sometimes remain unknown. What is known is that the cause of normal C1-Inh HAE is not related to reduced levels or dysfunction of the C1 inhibitor (in contrast to HAE types 1 and 2). Normal C1-Inh HAE can be diagnosed by reviewing the family history and noting that angioedema has been inherited from a previous generation (and thus it is hereditary angioedema). Normal C1-Inh HAE can also be diagnosed by determining that there is a dysfunction/fault/mutation in a gene other than those related to C1 inhibitor. For example, it has been reported that dysfunction/fault/mutation with plasminogen can cause normal C1-Inh HAE (see e.g. Veronez et al., Front Med (Lausanne). 2019 Feb 21;6:28. doi: 10.3389/fmed.2019.00028; or Recke et al., Clin Transl Allergy. 2019 Feb 14;9:9. doi: 10.1186/s13601-019-0247-x.). It has also been reported that dysfunction/fault/mutation with Factor XII can cause normal C1-Inh HAE (see e.g. Mansi et al. 2014 The Association for the Publication of the Journal of Internal Medicine Journal of Internal Medicine, 2015, 277; 585-593; or Maat et al. J Thromb Haemost. 2019 Jan;17(1):183-194. doi: 10.1111/jth.14325).

Acute HAE attacks normally progress through three key clinically distinct stages: an initial prodromal stage (that can typically last for up to 12 hours), followed by a swelling stage, and then an absorption stage. A majority of HAE attacks announce themselves with prodromal symptoms. Two thirds of prodromes appeared less than 6 hours before a HAE attack and no prodromes occur more than 24 hours before a HAE attack (Magerl et al. Clinical and Experimental Dermatology (2014) 39, pp298-303). For example, the following prodromal symptoms may start to be observed: a slight swelling (particularly affecting the face and neck), a typical type of abdominal pain, a typical reddening of the skin called "erythema marginatum". An attack is fully developed when it has reached maximum swelling and maximum expression of pain (e.g. abdominal attack), discomfort (e.g. peripheral attack) or threat to life (e.g. laryngeal attack). Once the attack has reached its peak, the subsequent time period to normalization is determined by the time it takes for the swelling to disappear and the liquid that has penetrated the tissues to be reabsorbed.

Synthetic and small molecule plasma kallikrein inhibitors have been described previously, for example by Garrett et al. ("Peptide aldehyde...." J. Peptide Res. 52, p62-71 (1998)), T. Griesbacher et al. ("Involvement of tissue kallikrein but not plasma kallikrein in the development of symptoms mediated by endogenous kinins in acute pancreatitis in rats" British Journal of Pharmacology 137, p692-700 (2002)), Evans ("Selective dipeptide inhibitors of kallikrein" WO03/076458), Szelke et al. ("Kininogenase inhibitors" WO92/04371), D. M. Evans et al. (Immunolpharmacology, 32, p115-116 (1996)), Szelke et al. ("Kininogen inhibitors" WO95/07921), Antonsson et al. ("New peptides derivatives" WO94/29335), J. Corte et al. ("Six membered heterocycles useful as serine protease inhibitors" WO2005/123680), J. Stürzbecher et al. (Brazilian J. Med. Biol. Res 27, p1929-34 (1994)), Kettner et al. (US 5,187,157), N. Teno et al. (Chem. Pharm. Bull. 41, p1079-1090 (1993)), W. B. Young et al. ("Small molecule inhibitors of plasma kallikrein" Bioorg. Med. Chem. Letts. 16, p2034-2036 (2006)), Okada et al. ("Development of potent and selective plasmin and plasma kallikrein inhibitors and studies on the structure-activity relationship" Chem. Pharm. Bull. 48, p1964-72 (2000)), Steinmetzer et al. ("Trypsin-like serine protease inhibitors and their preparation and use" WO08/049595), Zhang et al. ("Discovery of highly potent small molecule kallikrein inhibitors" Medicinal Chemistry 2, p545-553 (2006)), Sinha et al. ("Inhibitors of plasma kallikrein" WO08/016883), Shigenaga et al. ("Plasma Kallikrein Inhibitors" WO2011/118672), and Kolte et al. ("Biochemical characterization of a novel high-affinity and specific kallikrein inhibitor", British Journal of Pharmacology (2011), 162(7), 1639-1649). Also, Steinmetzer et al. ("Serine protease inhibitors" WO2012/004678) describes cyclized peptide analogs which are inhibitors of human plasmin and plasma kallikrein.

As explained above, HAE can manifest in patients who present with a genetic deficiency or dysfunction in C1 esterase inhibitor. Thus, some of the current treatments of HAE involve administering a C1 esterase inhibitor to normalise the deficiency or dysfunction in C1 esterase inhibitor. Such treatments can be prophylactic (*i.e*. administered in the absence of acute HAE attack symptoms to prevent/reduce the likelihood of an acute HAE attack) and/or acute treatments (*i.e*. administered when acute HAE attack symptoms are noticed to try to stop or reduce the severity of the acute HAE attack).

Cinryze^{®} and Haegarda^{®} contain a C1 esterase inhibitor and are indicated to prevent acute HAE attacks (*i.e*. prophylactic treatment). Treatment with Cinryze^{®} requires the preparation of a solution from a powder, which is then injected every 3 or 4 days. Similarly, treatment with Haegarda^{®} requires the preparation of a solution from a powder, which is then injected twice a week. It is not always possible for a patient to self-administer these treatments, and if this is the case, the patient is required to visit a clinic for treatment. Thus, both of these prophylactic treatments suffer from high patient burden. Additionally, the FDA packet insert for Haegarda^{®} states that it "should not be used to treat an acute HAE attack", and therefore a patient may require additional therapy if a HAE attack develops.

Berinert^{®} and Ruconest^{®} contain a C1 esterase inhibitor and are indicated to treat acute HAE attacks. Both of these treatments also involve the preparation of an injectable solution followed by injection. This process can be burdensome on the patient, especially when the patient is suffering from an acute HAE attack. Self-administration of the dosage amount is also not always possible, and if it is not, administration of the drug can be substantially delayed thus increasing the severity of the acute HAE attack for the patient.

To date, the only selective plasma kallikrein inhibitors approved for medical use in the treatment of HAE are Kalbitor^{®} (active substance ecallantide) and Takhzyro^{®} (active substance lanadelumab). Both treatments are formulated as solutions for injection. Ecallantide is a large protein plasma kallikrein inhibitor that presents a risk of anaphylactic reactions. Indeed, the EU marketing authorisation application for Kalbitor^{®} has recently been withdrawn because the benefits of Kalbitor^{®} are said to not outweigh its risks. Lanadelumab is a recombinant fully human IgG1 kappa light chain monoclonal antibody. Reported adverse reactions of treatment with lanadelumab include hypersensitivity, injection site pain, injection site erythema, and injection site bruising. The authorised EMA label for Takhzyro^{®} (active substance lanadelumab) states that it "is not intended for treatment of acute HAE attacks" and that "in case of a breakthrough HAE attack, individualized treatment should be initiated with an approved rescue medication". Also, as injections, both of these treatments involve a high patient burden.

Berotralstat (BCX7353) is being investigated as a once-daily oral treatment for the prevention of HAE attacks. Hwang et al. (Immunotherapy (2019) 11(17), 1439-1444) states that higher doses were associated with more gastrointestinal adverse effects indicating increased toxicity at higher levels.

Other plasma kallikrein inhibitors known in the art are generally small molecules, some of which include highly polar and ionisable functional groups, such as guanidines or amidines. Recently, plasma kallikrein inhibitors that do not feature guanidine or amidine functionalities have been reported. For example Brandl et al. ("N-((6-amino-pyridin-3-yl)methyl)-heteroaryl-carboxamides as inhibitors of plasma kallikrein" WO2012/017020), Evans et al. ("Benzylamine derivatives as inhibitors of plasma kallikrein" WO2013/005045), Allan et al. ("Benzylamine derivatives" WO2014/108679), Davie et al. ("Heterocyclic derivates" WO2014/188211), and Davie et al. ("N-((het)arylmethyl)-heteroaryl-carboxamides compounds as plasma kallikrein inhibitors" WO2016/083820).

The applicant has developed a novel series of compounds that are inhibitors of plasma kallikrein, which are disclosed in WO2016/083820 (PCT/GB2015/053615). These compounds demonstrate good selectivity for plasma kallikrein. One such compound is N-[(3-fluoro-4-methoxypyridin-2-yl)methyl]-3-(methoxymethyl)-1-({4-[(2-oxopyridin-1-yl)methyl]phenyl}methyl)pyrazole-4-carboxamide. The name N-[(3-fluoro-4-methoxypyridin-2-yl)methyl]-3-(methoxymethyl)-1-({4-[(2-oxopyridin-1-yl)methyl]phenyl}methyl)pyrazole-4-carboxamide denotes the structure depicted in Formula A.

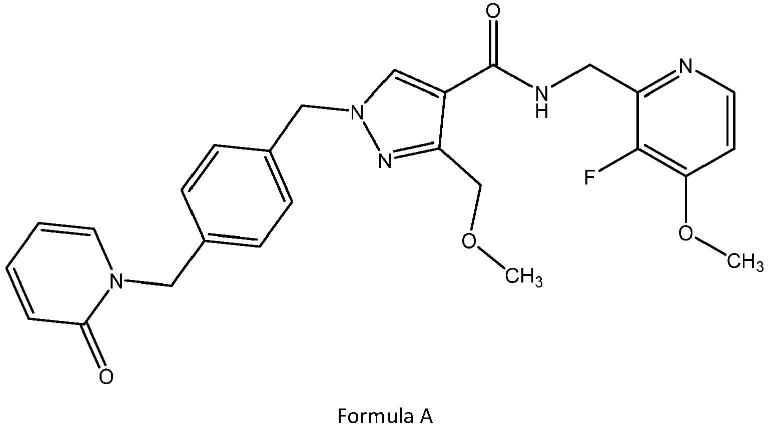

WO2019/106361 relates to oral solid dosage forms comprising a plasma kallikrein inhibitor, in particular a solid form (Form 1) of the compound of Formula A.

There is therefore the need for a treatment of HAE that is less burdensome on the patient to improve patient compliance. In particular, there is a need for a treatment of HAE that can be administered orally. There is also a need for an oral treatment of acute HAE attacks upon demand e.g. upon recognition of symptoms of an acute HAE attack. There is also a need for a prophylactic treatment of HAE to reduce the likelihood of an acute HAE attack. There is also a need for a treatment of acute HAE attacks that can be used on-demand by the patient and does not require regular (or continuous) dosing e.g. a treatment that does not require injections twice a week.

### Description of the Invention

The invention provides a compound of Formula A (or a pharmaceutically acceptable salt and/or solvate thereof) for use in treating hereditary angioedema (HAE) on-demand comprising: orally administering the compound of Formula A (or a pharmaceutically acceptable salt and/or solvate thereof) to a patient in need thereof on-demand,

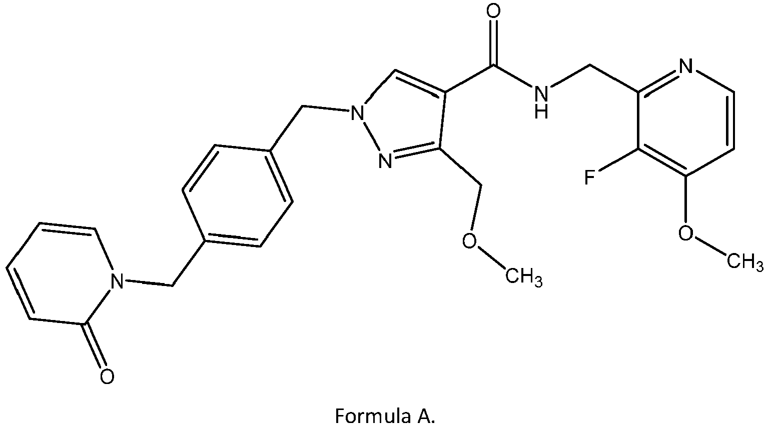

To date, there are no authorised on-demand oral treatments of HAE, with all of the authorised treatments being injectable. HAE attacks resolve faster and are shorter after early treatment (Maurer M et al. PLoS ONE 2013;8(2): e53773. doi:10.1371/journal.pone.0053773) and thus early intervention when an attack is expected, or ongoing, is essential to desirably manage the disease. Injectable treatments suffer from late dosing because the patient may need to prepare the dosage form or even travel to hospital for treatment. Therefore, HAE treatment is often undermined by late dosing caused by the high burden on the patient. Indeed, Maurer M et al. explains that more than 60% of patients administer their HAE injectable more than one hour after the onset of an attack. Without wishing to be bound by theory, it is thought that HAE injectable treatments suffer from late dosing for reasons such as inconvenience (self-administration is not always possible), pain (both during and after the injection), and hope (rather than treat, patients frequently will just hope for a less severe attack). The present invention aims to solve this problem.

The present invention provides a compound of Formula A for use in the treatment of HAE that is improved compared to any HAE treatment currently available. The present invention provides a compound of Formula A for use in the oral treatment of HAE that is particularly useful as an on-demand treatment of acute HAE attacks, and/or as an on-demand treatment to reduce the likelihood of an acute HAE attack. Specifically, as described herein, the compound of Formula A for use in treatments according to the invention (i) have a rapid onset of action, (ii) are potent, (iii) have a good safety profile, and (iv) have prolonged pharmacodynamic effects.

There is provided the compound of Formula A (or a pharmaceutically acceptable salt and/or solvate thereof) for use in treating hereditary angioedema (HAE) on-demand comprising: orally administering the compound of Formula A (or a pharmaceutically acceptable salt and/or solvate thereof) to a patient in need thereof on-demand.

In any of the treatments of the invention described herein, the term "compound of Formula A" is shorthand for "compound of Formula A (or a pharmaceutically acceptable salt and/or solvate thereof)". The term "solvate" is used herein to describe a molecular complex comprising the compound of the invention and a one or more pharmaceutically acceptable solvent molecules, for example, ethanol or water. The term "hydrate" is employed when the solvent is water and for the avoidance of any doubt, the term "hydrate" is encompassed by the term "solvate".

In any of the treatments of the invention described herein, the term "pharmaceutically acceptable salt" means a physiologically or toxicologically tolerable salt and includes, when appropriate, pharmaceutically acceptable base addition salts and pharmaceutically acceptable acid addition salts. For example (i) where a compound for use according to the invention contains one or more acidic groups, for example carboxy groups, pharmaceutically acceptable base addition salts that can be formed include sodium, potassium, calcium, magnesium and ammonium salts, or salts with organic amines, such as, diethylamine, N methylglucamine, diethanolamine or amino acids (e.g. lysine) and the like; (ii) where a compound for use according to the invention contains a basic group, such as an amino group, pharmaceutically acceptable acid addition salts that can be formed include hydrochlorides, hydrobromides, sulfates, phosphates, acetates, citrates, lactates, tartrates, mesylates, succinates, oxalates, phosphates, esylates, tosylates, benzenesulfonates, naphthalenedisulphonates, maleates, adipates, fumarates, hippurates, camphorates, xinafoates, p-acetamidobenzoates, dihydroxybenzoates, hydroxynaphthoates, succinates, ascorbates, oleates, bisulfates and the like.

Hemisalts of acids and bases can also be formed, for example, hemisulfate and hemicalcium salts.

For a review of suitable salts, see "Handbook of Pharmaceutical Salts: Properties, Selection and Use" by Stahl and Wermuth (Wiley-VCH, Weinheim, Germany, 2002).

The skilled person would understand "on-demand" treatment, in the context of HAE, to mean that the compound of Formula A is administered upon need of therapy in connection with one specific acute HAE attack. As described herein, this one specific HAE attack can be ongoing (e.g. treatment is initiated upon recognition of a symptom of an acute HAE attack) or likely to occur (e.g. when the patient anticipates that an acute HAE attack might be induced or triggered). Multiple dosage amounts of the compound of Formula A may be administered as part of the on-demand treatment, but these multiple dosages will be administered in connection with the same single acute HAE attack. In other words, "on-demand" does not require the administration of the compound of Formula A continuously at regular intervals (e.g. once a week, twice a week, etc.) irrespective of an instance of an acute HAE attack. This is in contrast to some other known treatments of HAE (e.g. treatments with Cinryze^{®} and Haegarda^{®}, as described above) that require continuous and regular dosing for therapy. Instead, in treatments of the invention, the compound of Formula A is taken when the patient requires fast-acting therapeutic effects. Particular "on-demand" treatments of the invention include: (i) treating an acute attack of HAE on-demand, when the compound of Formula A is administered upon recognition of a symptom of an acute HAE attack, and (ii) prophylactically reducing the likelihood of an HAE attack on-demand, e.g. when it is anticipated that an acute HAE attack might be induced (or triggered). These are discussed below in more detail.

In any of the treatments of the invention described herein, the patient is preferably a human. HAE is a hereditary disease and patients of all ages can suffer from HAE attacks. Accordingly, the human patient can be a child (ages 0 to 18 years) or an adult (18 years old or older). Specifically, the patient can be aged 12 years and above. The patient can also be aged 2 years and above.

As demonstrated in the examples, the compound of Formula A is a potent inhibitor of plasma kallikrein. As already explained, inhibiting plasma kallikrein inhibits the cleavage of high molecular weight kininogen that contributes to an HAE attack. Additionally, and as demonstrated in Example 4, the compound of Formula A is also capable of reducing the cleavage of plasma prekallikrein and the generation of Factor XIIa (FXlla) following activation of the contact system. These advantageous additional effects support the treatment of the invention being highly efficacious and are demonstrated in particular when the concentration of the compound of Formula A is at least 500 ng/mL of plasma. A plasma concentration of at least 500 ng/mL can be observed following administration of a dosage amount of at least about 60 mg (more specifically, at least about 70 or about 80 mg) of the compound of Formula A.

Accordingly, in any of the treatments of the invention disclosed herein, particularly following a dosage amount of the compound of Formula A of at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg), in addition to inhibiting plasma kallikrein, the treatments can also reduce the cleavage of plasma prekallikrein to generate plasma kallikrein and/or reduce the generation of Factor XIIa (FXlla) following administration. Thus, in some embodiments, particularly following a dosage amount of the compound of Formula A of at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg), the treatments can block the cleavage of plasma prekallikrein to generate plasma kallikrein and/or block the cleavage of FXII to generate FXlla.

The terms "acute attack of HAE" and "acute HAE attack" are used interchangeably herein. The term "hereditary angioedema" means any bradykinin-mediated angioedema caused by an inherited genetic dysfunction, fault, or mutation. As a result, the term "HAE" includes at least HAE type 1, HAE type 2, and normal C1 inhibitor HAE (normal C1-Inh HAE).

### The compound of Formula A for use in treatments of acute HAE attacks on-demand

An aspect of the invention provides the compound of Formula A for use in treating an acute attack of hereditary angioedema (HAE) on-demand comprising: orally administering the compound of Formula A to a patient in need thereof, wherein the compound of Formula A is orally administered on-demand upon recognition of a symptom of an acute HAE attack.

Although each HAE attack can be different in severity and in terms of the area affected, patients who suffer from HAE, medical professionals with knowledge of HAE, and carers of HAE patients are (and indeed the skilled person would be) astute in identifying symptoms of an acute HAE attack. These symptoms include, but are not limited to: swelling of tissues such as in the hands, feet, limbs, face, intestinal tract, and/or airway; fatigue; headache; muscle aches; skin tingling; abdominal pain; nausea; vomiting; diarrhoea; difficulty swallowing; hoarseness; shortness of breath; and/or mood changes. Thus, in some embodiments, administration of the compound of Formula A can occur upon recognition of at least one of the above symptoms.

The skilled person would also understand that "administered upon recognition of a symptom of a HAE attack" means that administration occurs as quickly as feasibly possible after the symptom of an acute HAE attack is recognised. For example, patients are expected to have the compound of Formula A easily and readily available at all times (most likely in the form of a pharmaceutically acceptable composition) to ensure that treatment can occur upon recognition of a symptom of a HAE attack. In other words, the treatment occurs on-demand. For example, in some embodiments, the compound of Formula A for use according to the invention can be administered within 1 hour of the symptom of an acute HAE attack being recognised, preferably within 30 minutes, within 20 minutes, within 10 minutes, or within 5 minutes of the symptom of an acute HAE attack being recognised.

If the symptom of an acute HAE attack is recognised in the prodromal phase, an embodiment of the invention is that the compound of Formula A for use according to the invention can be administered in the prodromal phase of an acute HAE attack. In these circumstances, the symptom recognised can be a slight swelling, in particular, a slight swelling affecting the face and neck. In addition, or in the alternative, the symptom can be abdominal pain, in particular, abdominal pain is considered to be characteristic of a HAE attack. In addition, or in the alternative, the symptom can be a reddening of the skin such as erythema marginatum.

The compound of Formula A for use in accordance with the invention can prevent an acute HAE attack from increasing in severity. In some circumstances, treatment can shorten the attack duration, and sometimes even halt the attack in its entirety. For instance, treatment can halt the progression of a peripheral HAE attack or an abdominal HAE attack. In some embodiments, the compound of Formula A for use according to the invention can suppress the subsequent onset of swelling, sometimes completely, and in particular when treatment is initiated in the prodromal phase. In particular, in some embodiments, the acute HAE attack can be prevented from progressing into the swelling stage when the treatment is initiated in the prodromal phase.

The compound of Formula A can be sufficient for treating the acute HAE attack alone *i.e*. without the patient being administered any active pharmaceutical ingredient other than the compound of Formula A.

Thus, in some embodiments of the invention, no active pharmaceutical ingredient other than the compound of Formula A is administered to the patient in order to treat the acute HAE attack. In particular, in some embodiments, the compound of Formula A for use in the treatments of the invention do not require administering any active pharmaceutical ingredient for treating an HAE attack (e.g. a rescue medication such as pdC1INH, rhC1INH, or icatibant) other than the compound of Formula A. More specifically, in some embodiments, no active pharmaceutical ingredient for treating an HAE attack (e.g. a rescue medication such as pdC1INH, rhC1INH, or icatibant) other than the compound of Formula A is administered to the patient.

Alternatively, in some embodiments, the compound of Formula A for use in the treatments of the invention may be used in combination with other treatments of HAE. For example, in some embodiments, the compound of Formula A for use in the on-demand acute therapy described herein can be used as a "top-up" to another treatment of HAE. In some embodiments, the patient may be taking another prophylactic treatment of HAE and might use the compound of Formula A for use in the on-demand treatments described herein to treat an acute HAE attack that was not prevented by the other prophylactic treatment of HAE.

For instance, in some embodiments, the compound of Formula A for use in a method for treating HAE in a patient already taking a C1 inhibitor (such as Cinryze^{®}, Haegarda^{®}, Berinert^{®}) for prophylaxis is provided comprising: orally administering the compound of Formula A to the patient on-demand upon recognition of a symptom of an acute HAE attack. In another embodiment, the compound of Formula A for use in a method for treating HAE in a patient already taking lanadelumab for prophylaxis is provided comprising: orally administering the compound of Formula A to the patient on-demand upon recognition of a symptom of an acute HAE attack. In another embodiment, the compound of Formula A for use in a method for treating HAE in a patient already taking berotralstat for prophylaxis is provided comprising: orally administering the compound of Formula A to the patient on-demand upon recognition of a symptom of an acute HAE attack.

In any of the above treatments, the symptom can be recognised by the patient. In any of the above treatments, the symptom can be recognised by a medical professional such as a medical professional with knowledge of HAE. In any of the above treatments, the symptom can be recognised by a carer of the patient.

The compound of Formula A for use in treatments according to the invention can reduce the proportion of HAE attacks that progress by one level or more on a 5-point Likert scale (5LS). The compound of Formula A for use in treatments according to the invention can reduce the proportion of HAE attacks that progress by one level or more on a 5LS within 12 hours of administering the compound. The compound of Formula A for use in treatments according to the invention can improve the resolution time of a HAE attack to "none" on a 5LS. 5LS is a known scale in the art (see e.g. Allergy Asthma Proc. 2018 Jan 1;39(1):74-80. doi: 10.2500/aap.2018.39.4095) that can be used to report the severity of HAE attacks and for example can be used to report attacks as "none", "mild", "moderate", "severe" or "very severe".

The compound of Formula A for use in treatments according to the invention can reduce the proportion of HAE attacks that are rated "worse" or "much worse" on a 7-point transition question (7TQ). The compound of Formula A for use in treatments according to the invention can increase the proportion of HAE attacks that are rated as "better" or "much better". 7TQ is a known index in the art that can be used to score the progression of an HAE attack and to report attacks as "much better", "better", "a little better", "no change", "a little worse", "worse", or "much worse".

In some embodiments of any of the on-demand treatments of an acute HAE attack of the invention, the patient can be administered a single dosage amount of the compound of Formula A to treat the acute HAE attack. In some other embodiments of any of the on-demand treatments of acute HAE attacks of the invention, the patient can be administered multiple dosage amounts of the compound of Formula A to treat the acute HAE attack. For example, the on-demand treatment can comprise administering two dosage amounts of the compound of Formula A within a 24 hour period starting from the time of taking the first dosage amount. Alternatively, the on-demand treatment can comprise administering three dosage amounts of the compound of Formula A within a 24 hour period starting from the time of taking the first dosage amount. Alternatively, the on-demand treatment can comprise administering four dosage amounts of the compound of Formula A within a 24 hour period starting from the time of taking the first dosage amount. When multiple dosage amounts are taken, the dosage amount can be evenly spaced apart such that there is an approximately equal time period between each dosage amount e.g. taking the subsequent dosage amount at 8 hours, 16 hours and 24 hours following the first dosage amount.

In some embodiments of any of the on-demand treatments of an acute HAE attack of the invention, the patient can be administered the daily dosage amount in two dosage amounts per day. These two dosage amounts can be administered simultaneously, separately or sequentially. In some embodiments, the two dosage amounts can be administered at any time within the day, with the interval between the two dosage amounts being specific to the patient, and the severity of the acute HAE attack. In some embodiments, the second dosage amount can be administered within about 2 hours of the first (more specifically, between about 1 and 2 hours following the first dosage amount). In some embodiments, the second dosage amount can be administered between about 1 and about 4 hours of the first (more specifically, between about 1 and 3 hours, about 2 and 3 hours, or between 3 hours and about 4 hours, following the first dosage amount). In some embodiments, the second dosage amount can be administered between about 4 and about 12 hours of the first (more specifically, between about 4 and about 8 hours, or at about 6 hours, following the first dosage amount). In some embodiments, the second dosage amount can be administered between about 2 and about 6 hours of the first (more specifically, between about 3 and about 6 hours, following the first dosage amount). In some embodiments, the second dosage amount can be administered within about 8 hours of the first (more specifically, between about 4 and about 8 hours following the first dosage amount). In some embodiments, the second dosage amount can be administered within about 12 hours of the first (more specifically, between about 8 and about 12 hours following the first dosage amount). In some embodiments, the second dosage amount can be administered within about 16 hours of the first (more specifically, between about 12 and about 16 hours following the first dosage amount). In some embodiments, the second dosage amount can be administered within about 20 hours of the first (more specifically, between about 16 and about 20 hours following the first dosage amount). In some embodiments, the second dosage amount can be administered within about 24 hours of the first (more specifically, between about 20 and about 24 hours following the first dosage amount). In these embodiments, each of the two dosage amounts can be 600 mg of the compound of Formula A.

In any of the on-demand treatments of an acute HAE attack of the invention, the patient can be administered the daily dosage amount in two dosage amounts per day, wherein the second dosage amount can be administered at least about 6 hours after the first dosage amount. The patient can be administered the daily dosage amount in two dosage amounts per day, wherein the second dosage amount can be administered between about 5 and about 7 hours after the first dosage amount. More specifically, the patient can be administered the daily dosage amount in two dosage amounts per day, wherein the second dosage amount can be administered about 6 hours after the first dosage amount. In these embodiments, each of the two dosage amounts can be 600 mg of the compound of Formula A. Each of these 600 mg dosage amounts can be two tablets comprising 300 mg of the compound of Formula A.

In some embodiments of any of the on-demand treatments of an acute HAE attack of the invention, the patient can be administered the daily dosage amount in three dosage amounts per day. These three dosage amounts can be administered simultaneously, separately or sequentially. In some embodiments, the three dosage amounts can be administered at any time within the day, with the interval between the three dosage amounts being specific to the patient, and the severity of the acute HAE attack. In some embodiments, the second and third dosage amounts can be both administered within about 4 hours of the first. More specifically, the second dosage amount can be administered between about 1 and 3 hours following the first dosage amount and the third dosage amount can be administered between about 3 and about 4 hours following the first dosage amount. The second dosage amount can be administered between about 4 and about 12 hours of the first (more specifically, between about 4 and about 8 hours, or at about 6 hours, following the first dosage amount), and the third dosage amount can be administered between about 4 and about 12 hours of the second (more specifically, between about 4 and about 8 hours, or at about 6 hours, following the second dosage amount). Even more specifically, the second dosage amount can be administered about 2 hours following the first dosage amount and the third dosage amount can be administered about 4 hours following the first dosage amount. In some embodiments, the second and third dosage amounts can both be administered within about 8 hours of the first. More specifically, the second dosage amount can be administered between about 3 and 5 hours of the first dosage amount and the third dosage amount can be administered between about 7 and about 8 hours following the first dosage amount. Even more specifically, the second dosage amount can be administered about 4 hours following the first dosage amount and the third dosage amount can be administered about 8 hours following the first dosage amount. In some embodiments, the second and third dosage amounts can both be administered within about 16 hours of the first. More specifically, the second dosage amount can be administered between about 7 and 9 hours of the first dosage amount and the third dosage amount can be administered between about 15 and about 16 hours following the first dosage amount. Even more specifically, the second dosage amount can be administered about 8 hours following the first dosage amount and the third dosage amount can be administered about 16 hours following the first dosage amount. In these embodiments, each of the three dosage amounts can be 600 mg of the compound of Formula A.

In any of the on-demand treatments of an acute HAE attack of the invention, the patient can be administered the daily dosage amount in three dosage amounts per day, wherein the second and third dosage amounts can be administered at least about 6 hours after the preceding dosage amount. The patient can be administered the daily dosage amount in three dosage amounts per day, wherein the second dosage amount can be administered between about 5 and about 7 hours after the first dosage amount, and the third dosage amount can be administered between about 11 and about 13 hours after the first dosage amount. More specifically, the patient can be administered the daily dosage amount in three dosage amounts per day, wherein the second dosage amount can be administered about 6 hours after the first dosage amount and the third dosage amount can be administered about 12 hours after the first dosage amount. In these embodiments, each of the three dosage amounts can be 600 mg of the compound of Formula A. Each of these 600 mg dosage amounts can be two tablets comprising 300 mg of the compound of Formula A.

Multiple dosage amounts can be administered if, for example, a HAE attack persists after administration of the first dosage amount. When used in this context, "persists" can mean that, e.g., the first dosage amount does not prevent an acute HAE attack from increasing in severity, or that the first dosage amount does not halt the HAE attack in its entirety, or that the first dosage amount does not decrease the severity of the HAE attack. Accordingly, the compound of Formula A for use in on-demand treatments of an HAE attack of the invention can comprise administering a first dosage amount, and then administering a second dosage amount if the HAE attack persists after administering the first dosage amount. The compound of Formula A for use in on-demand treatments of an HAE attack of the invention can also comprise administering a first dosage amount, and then administering a second dosage amount if the HAE attack persists after administering the first dosage amount, and then administering a third dosage amount if the HAE attack persists after administering the second dosage amount. In each case, each subsequent dosage amount can be administered simultaneously, separately or sequentially. In each case, each subsequent dosage amount can be administered at least about 6 hours (e.g. at about 6 hours) after the preceding dosage amount. In each case, each dosage amount can comprise 600 mg of the compound, e.g., administered as two tablets comprising 300 mg.

Specifically, the compound of Formula A for use in the on-demand treatments of an acute HAE attack of the invention can comprise administering a first dosage amount comprising 600 mg of the compound (e.g. as two tablets comprising 300 mg of the compound), and then administering a second dosage amount comprising 600 mg of the compound (e.g. as two tablets comprising 300 mg of the compound) if the HAE attack persists after administering the first dosage amount. The second dosage amount can be administered at least about 6 hours (e.g. at about 6 hours) after the first dosage amount. If the HAE attack persists after the second dosage amount, the on-demand treatments of an acute HAE attack of the invention can comprise administering a third dosage amount comprising 600 mg of the compound (e.g. as two tablets comprising 300 mg of the compound). The third dosage amount can be administered at least about 6 hours (e.g. at about 6 hours) after the second dosage amount.

Multiple dosage amounts can be administered even if the severity of the HAE attack appears to have been reduced (or even halted in its entirety) after administration of the first dosage amount to prevent the HAE attack from increasing in severity again. For example, multiple dosage amounts can be used for the peace of mind of the patient e.g. to ease anxiety of the patient. Accordingly, the compound of Formula A for use in on-demand treatments of an HAE attack of the invention can comprise administering a first dosage amount, and then administering a second dosage amount even if the severity of the HAE attack appears to have been reduced (or even halted in its entirety) after administration of the first dosage amount to prevent the HAE attack from increasing in severity again. Even if the severity of the HAE attack appears to have been reduced (or even halted in its entirety) after administration of the first and/or second dosage amount, the compound of Formula A for use in on-demand treatments of an HAE attack of the invention can also comprise administering a third dosage amount to prevent the HAE attack from increasing in severity again. In each case, each subsequent dosage amount can be administered simultaneously, separately or sequentially. In each case, each subsequent dosage amount can be administered at least about 6 hours (e.g. at about 6 hours) after the preceding dosage amount. In each case, each dosage amount can comprise 600 mg of the compound, e.g., administered as two tablets comprising 300 mg of the compound.

Specifically, the compound of Formula A for use in the on-demand treatments of an acute HAE attack of the invention can comprise administering a first dosage amount comprising 600 mg of the compound (e.g. as two tablets comprising 300 mg of the compound), and then administering a second dosage amount comprising 600 mg of the compound (e.g. as two tablets comprising 300 mg of the compound) even if the severity of the HAE attack appears to have been reduced (or even halted in its entirety) after administration of the first dosage amount to prevent the HAE attack from increasing in severity again. The second dosage amount can be administered at least about 6 hours (e.g. at about 6 hours) after the first dosage amount. Even if the severity of the HAE attack appears to have been reduced (or even halted in its entirety) after administration of the first and/or second dosage amount, the compound of Formula A for use in the on-demand treatments of an acute HAE attack of the invention can comprise administering a third dosage amount comprising 600 mg of the compound (e.g. as two tablets comprising 300 mg of the compound) to prevent the HAE attack from increasing in severity again. The third dosage amount can be administered at least about 6 hours (e.g. at about 6 hours) after the second dosage amount.

The compound of Formula A for use in the on-demand treatments of an acute HAE attack of the invention can comprise not administering more than three dosage amounts in a 24 hour period (e.g. three dosage amounts comprising 600 mg of the compound, optionally as 6 tablets each comprising 300 mg of the compound).

### The compound of Formula A for use in on-demand prophylactic treatment of acute HAE attacks

An aspect of the invention provides the compound of Formula A for use in treating hereditary angioedema (HAE) comprising: orally administering the compound of Formula A to a patient in need thereof, wherein the compound of Formula A is orally administered on-demand to prophylactically reduce the likelihood of an acute HAE attack.

In some embodiments, the compound of Formula A can be administered to prevent an acute HAE attack.

As discussed above, the compound of Formula A for use in accordance with the invention do not require dosing of the compound of Formula A at regular intervals to provide prophylactic therapy. Indeed, the compound of Formula A is administered on-demand. For example, the compound of Formula A can be administered on-demand to reduce the likelihood of an acute HAE attack (e.g. to prevent an acute HAE attack) when it is anticipated that an acute HAE attack will be induced (or triggered) i.e. it is anticipated that the patient will suffer from an acute HAE attack. In some embodiments, the patient can anticipate than an acute HAE attack will be induced (or triggered). In some embodiments, a medical professional such as a medical professional with knowledge of HAE can anticipate than an acute HAE attack will be induced (or triggered). In some embodiments, a carer for the patient can anticipate than an acute HAE attack will be induced (or triggered). For example, an acute HAE attack can be induced (or triggered) by various stimuli such as physical traumata (e.g. medical, dental or surgical procedures) and/or stress (e.g. high stress situations such as mental stress, which in some instances can be associated with taking examinations or mental stress associated with a medical, dental or surgical procedure). For example, an acute HAE attack can be induced (or triggered) by the elevated stress/anxiety levels of the patient when the patient might expect to have an HAE attack. Additionally, the frequency of acute HAE attacks instances can vary over time in the same patient. Patients can often suffer from periods where the frequency of instances of acute HAE attacks is greater than normal. So, an acute HAE attack can be anticipated during periods where the patient is suffering from more frequent instances of acute HAE attacks compared to normal. Those familiar with HAE will be aware that acute HAE attacks can be induced (or triggered) in this way. Patients, medical professionals with knowledge of HAE, and carers of patients can also be astute in anticipating such triggers. Thus, in accordance with the invention, the compound of Formula A can be administered on-demand when it is anticipated that the patient will be subjected to one or more of these stimuli or circumstances.

As discussed above, the patient can be administered the compound of Formula A as part of an on-demand prophylactic treatment of an acute HAE attack. As discussed above, this treatment reduces the likelihood of an acute HAE attack. However, in some circumstances, a patient can still suffer from an acute HAE attack. Thus, an embodiment of the invention is that the patient can be administered the compound of Formula A as part of an on-demand prophylactic treatment of an acute HAE attack, as discussed above, further comprising taking an on-demand dosage amount of the compound of Formula A upon recognition of a symptom of an acute HAE attack to treat an acute HAE attack should it arise. These on-demand treatments of acute HAE attacks are discussed above.

Thus, in some embodiments, there is provided the compound of Formula A for use in a method for treating hereditary angioedema (HAE) on-demand comprising: orally administering the compound of Formula A to a patient in need thereof, wherein the compound of Formula A is orally administered on-demand to prophylactically reduce the likelihood of an acute HAE attack, further comprising orally administering the compound of Formula A on-demand upon recognition of a symptom of an acute HAE attack.

In some embodiments of any of the on-demand treatments of an acute HAE attack of the invention, the patient can be administered a single dosage amount of the compound of Formula A to treat the acute HAE attack. In some other embodiments of any of the on-demand treatments of acute HAE attacks of the invention, the patient can be administered multiple dosage amounts of the compound of Formula A to treat the acute HAE attack. For example, the on-demand treatment can comprise administering two dosage amounts of the compound of Formula A within a 24 hour period starting from the time of taking the first dosage amount. Alternatively, the on-demand treatment can comprise administering three dosage amounts of the compound of Formula A within a 24 hour period starting from the time of taking the first dosage amount. Alternatively, the on-demand treatment can comprise administering four dosage amounts of the compound of Formula A within a 24 hour period starting from the time of taking the first dosage amount. When multiple dosage amounts are taken, the dosage amount can be evenly spaced apart such that there is an approximately equal time period between each dosage amount e.g. taking the subsequent dosage amount at 8 hours, 16 hours and 24 hours following the initial dosage amount.

In some embodiments of any of the on-demand prophylactic treatments of acute HAE attacks described herein, the patient can be administered two dosage amounts per day. These two dosage amounts can be administered simultaneously, separately or sequentially. In some embodiments, the two dosage amounts can be administered at any time within the day, with the interval between the two dosage amounts being specific to the patient. In some embodiments, the second dosage amount can be administered within about 2 hours of the first (more specifically, between about 1 and 2 hours following the first dosage amount). In some embodiments, the second dosage amount can be administered between about 1 and about 4 hours of the first (more specifically, between about 1 and 3 hours, about 2 and 3 hours, or between 3 hours and about 4 hours, following the first dosage amount). In some embodiments, the second dosage amount can be administered between about 4 and about 12 hours of the first (more specifically, between about 4 and about 8 hours, or at about 6 hours, following the first dosage amount). In some embodiments, the second dosage amount can be administered between about 2 and about 6 hours of the first (more specifically, between about 3 and about 6 hours, following the first dosage amount). In some embodiments, the second dosage amount can be administered within about 8 hours of the first (more specifically, between about 4 and about 8 hours following the first dosage amount). In some embodiments, the second dosage amount can be administered within about 12 hours of the first (more specifically, between about 8 and about 12 hours following the first dosage amount). In some embodiments, the second dosage amount can be administered within about 16 hours of the first (more specifically, between about 12 and about 16 hours following the first dosage amount). In some embodiments, the second dosage amount can be administered within about 20 hours of the first (more specifically, between about 16 and about 20 hours following the first dosage amount). In some embodiments, the second dosage amount can be administered within about 24 hours of the first (more specifically, between about 20 and about 24 hours following the first dosage amount). In these embodiments, each of the two dosage amounts can be 600 mg of the compound of Formula A.

In any of the on-demand prophylactic treatments of acute HAE attacks described herein, the patient can be administered the daily dosage amount in two dosage amounts per day, wherein the second dosage amount can be administered at least about 6 hours after the first dosage amount. The patient can be administered the daily dosage amount in two dosage amounts per day, wherein the second dosage amount can be administered between about 5 and about 7 hours after the first dosage amount. More specifically, the patient can be administered the daily dosage amount in two dosage amounts per day, wherein the second dosage amount can be administered about 6 hours after the first dosage amount. In these embodiments, each of the two dosage amounts can be 600 mg of the compound of Formula A. Each of these 600 mg dosage amounts can be two tablets comprising 300 mg of the compound of Formula A.

In some embodiments of any of the on-demand prophylactic treatments of acute HAE attacks described herein, the patient can be administered the daily dosage amount in three dosage amounts per day. These three dosage amounts can be administered simultaneously, separately or sequentially. In some embodiments, the three dosage amounts can be administered at any time within the day, with the interval between the three dosage amounts being specific to the patient. In some embodiments, the second and third dosage amounts can be both administered within about 4 hours of the first. More specifically, the second dosage amount can be administered between about 1 and 3 hours following the first dosage amount and the third dosage amount can be administered between about 3 and about 4 hours following the first dosage amount. The second dosage amount can be administered between about 4 and about 12 hours of the first (more specifically, between about 4 and about 8 hours, or at about 6 hours, following the first dosage amount), and the third dosage amount can be administered between about 4 and about 12 hours of the second (more specifically, between about 4 and about 8 hours, or at about 6 hours, following the second dosage amount). Even more specifically, the second dosage amount can be administered about 2 hours following the first dosage amount and the third dosage amount can be administered about 4 hours following the first dosage amount. In some embodiments, the second and third dosage amounts can both be administered within about 8 hours of the first. More specifically, the second dosage amount can be administered between about 3 and 5 hours of the first dosage amount and the third dosage amount can be administered between about 7 and about 8 hours following the first dosage amount. Even more specifically, the second dosage amount can be administered about 4 hours following the first dosage amount and the third dosage amount can be administered about 8 hours following the first dosage amount. In some embodiments, the second and third dosage amounts can both be administered within about 16 hours of the first. More specifically, the second dosage amount can be administered between about 7 and 9 hours of the first dosage amount and the third dosage amount can be administered between about 15 and about 16 hours following the first dosage amount. Even more specifically, the second dosage amount can be administered about 8 hours following the first dosage amount and the third dosage amount can be administered about 16 hours following the first dosage amount. In these embodiments, each of the three dosage amounts can be 600 mg of the compound of Formula A.

In any of the on-demand prophylactic treatments of acute HAE attacks described herein, the patient can be administered the daily dosage amount in three dosage amounts per day, wherein the second and third dosage amounts can be administered at least about 6 hours after the preceding dosage amount. The patient can be administered the daily dosage amount in three dosage amounts per day, wherein the second dosage amount can be administered between about 5 and about 7 hours after the first dosage amount, and the third dosage amount can be administered between about 11 and about 13 hours after the first dosage amount. More specifically, the patient can be administered the daily dosage amount in three dosage amounts per day, wherein the second dosage amount can be administered about 6 hours after the first dosage amount and the third dosage amount can be administered about 12 hours after the first dosage amount. In these embodiments, each of the three dosage amounts can be 600 mg of the compound of Formula A. Each of these 600 mg dosage amounts can be two tablets comprising 300 mg of the compound of Formula A.

Multiple dosage amounts can be administered if, for example, there is a continued need to prophylactically reduce the likelihood of an acute HAE attack (e.g. if the patient continues to anticipate that a HAE attack might be induced, as discussed above). Accordingly, the compound of Formula A for use in on-demand treatments of an HAE attack of the invention can comprise administering a first dosage amount, and then administering a second dosage amount if there is a continued need to prophylactically reduce the likelihood of an acute HAE attack after administering the first dosage amount. The compound of Formula A for use in on-demand treatments of an HAE attack of the invention can also comprise administering a first dosage amount, and then administering a second dosage amount if there is a continued need to prophylactically reduce the likelihood of an acute HAE attack after administering the first dosage amount, and then administering a third dosage amount if there is a continued need to prophylactically reduce the likelihood of an acute HAE attack after administering the second dosage amount. In each case, each subsequent dosage amount can be administered simultaneously, separately or sequentially. In each case, each subsequent dosage amount can be administered at least about 6 hours (e.g. at about 6 hours) after the preceding dosage amount. In each case, each dosage amount can comprise 600 mg of the compound, e.g., administered as two tablets comprising 300 mg of the compound.

Specifically, the on-demand prophylactic treatments of acute HAE attacks described herein can comprise administering a first dosage amount comprising 600 mg of the compound (e.g. as two tablets each comprising 300 mg of the compound), and then administering a second dosage amount comprising 600 mg of the compound (e.g. as two tablets each comprising 300 mg of the compound) if there is a continued need to prophylactically reduce the likelihood of an acute HAE attack after administering the first dosage amount. The second dosage amount can be administered at least about 6 hours (e.g. at about 6 hours) after the first dosage amount. If there is a continued need to prophylactically reduce the likelihood of an acute HAE attack after the second dosage amount, the compound of Formula A for use in the on-demand treatments of an acute HAE attack of the invention can comprise administering a third dosage amount comprising 600 mg of the compound (e.g. as two tablets each comprising 300 mg of the compound). The third dosage amount can be administered at least about 6 hours (e.g. at about 6 hours) after the second dosage amount.

The on-demand prophylactic treatments of acute HAE attacks described herein can comprise not administering more than three dosage amounts in a 24 hour period (e.g. three dosage amounts comprising 600 mg of the compound, optionally as 6 tablets each comprising 300 mg of the compound).

As described above, the invention provides a compound of Formula A (or a pharmaceutically acceptable salt and/or solvate thereof) for use in treating hereditary angioedema (HAE) on-demand comprising: orally administering the compound of Formula A (or a pharmaceutically acceptable salt and/or solvate thereof) to a patient in need thereof on-demand,

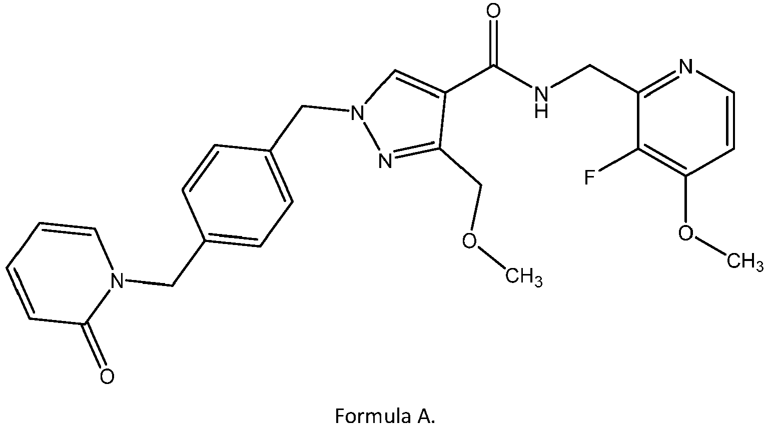

In some embodiments, the invention provides a compound of Formula A (or a pharmaceutically acceptable salt and/or solvate thereof) for use in treating hereditary angioedema (HAE) on-demand comprising: orally administering the compound of Formula A to a patient in need thereof, wherein the compound of Formula A is orally administered to prophylactically reduce the likelihood of an acute HAE attack, wherein the compound of Formula A is administered regularly to the patient, further comprising orally administering the compound of Formula A on-demand upon recognition of a symptom of an acute HAE attack.

The term "administered regularly" is intended to mean administering the compound of Formula A continuously at regular intervals (e.g. once a week, twice a week, etc.) to provide an effective treatment. The healthcare professional would readily understand what regular (or continuous) administration is intended to mean.

### Dosing

In any of the treatments of the invention described herein, the compound of Formula A is orally administered in a therapeutically effective amount.

In some embodiments, the compound of Formula A can be administered at a daily dosage amount of between about 5 mg and about 2000 mg per day. "Daily dosage amount" means the total amount administered in one day. More specifically, the compound of Formula A can be administered at a daily dosage amount of between about 100 mg and about 1500 mg, about 300 mg to about 1800 mg, about 100 mg and about 1400 mg, about 200 mg and about 1200 mg, about 300 mg and about 1200 mg, about 600 mg and about 1200 mg, about 450 mg and about 900 mg, about 500 mg and about 1000 mg, about 450 mg and about 600 mg, about 500 mg and about 700 mg (more specifically, 600 mg), about 800 mg and about 1000 mg per day, about 900 mg and about 1400 mg (more specifically 1200 mg), or about 900 mg and about 1200 mg. In a specific embodiment, the daily dosage amount is 300 mg. In another specific embodiment, the daily dosage amount is 600 mg. In another specific embodiment, the daily dosage amount is 900 mg. In another specific embodiment, the daily dosage amount is 1200 mg. In another specific embodiment the daily dosage amount is 1800 mg.

The daily dosage amount can be administered as one single dosage amount, or sub-divided into multiple dosage amounts for administration periodically during the day. In turn, each dosage amount can administered as a single dosage form, or sub-divided into multiple dosage forms. For example, a 1200 mg daily dosage amount can be administered as two sub-divided dosage amounts of 600 mg, where each of these sub-divided dosage amounts can be administered as two sub-divided dosage forms of 300 mg. Where multiple dosage amounts and multiple dosage forms are used, these can be administered simultaneously, separately or sequentially.

In some embodiments, each single unit dosage form comprising the compound of Formula A comprises between about 5 mg and about 1000 mg, about 50 mg to about 800 mg, about 100 mg to about 700 mg, about 200 mg to about 700 mg, about 300 mg to about 700 mg, or about 500 mg to about 700 mg of the compound of Formula A. In some embodiments, each single unit dosage form comprising the compound of Formula A comprises: about 5 mg, about 10 mg, about 20 mg, about 40, about 80 mg, about 160 mg, about 300 mg, about 400 mg, about 450 mg, about 500 mg or about 600 mg.

Each dosage amount administered to the patient can comprise 600 mg of the compound that may be sub-divided into two tablets comprising 300 mg of the compound.

Alternatively, each dosage amount can comprise 300 mg of the compound that may be one tablet comprising 300 mg of the compound.

In a specific embodiment, the patient is administered a daily dosage amount of 600 mg, which is administered as one dosage amount.

In another specific embodiment, the patient is administered a daily dosage amount of 1200 mg, which is administered as two dosage amounts, and in particular when the second dosage amount is administered between 2 and 6 hours of the first, preferably between about 3 and 6 hours of the first dosage amount.

In another specific embodiment, the patient is administered a daily dosage amount of 1800 mg, which is administered as three dosage amounts, and in particular when the second dosage amount is administered between 2 and 8 hours of the first (e.g. at about 2 hours, about 4 hours, about 6 hours, or about 8 hours), and the third dosage amount is administered between about 4 and 16 hours of the first dosage amount (e.g. at about 4 hours, about 6 hours, about 8 hours, about 12 hours, or about 16 hours).

The treatments of the invention involve oral administration. In any of the treatments of the invention, the compound of Formula A can be administered as an oral dosage form comprising the compound of Formula A and pharmaceutically acceptable excipients. The oral dosage form can be in the form of a tablet or a capsule. In one embodiment the oral dosage form is a tablet. In another embodiment, the oral dosage form is a capsule.

The compound of Formula A for use in the treatments of the invention can comprise not administering more than three dosage amounts in a 24 hour period. Specifically, if each dosage amount comprises 600 mg of the compound, this means that the compound of Formula A for use in the treatments of the inventions can comprise not administering more than 1800 mg of the compound in a 24 hour period. If each dosage amount comprising 600 mg of the compound is sub-divided into two dosage amounts (e.g. tablets) comprising 300 mg of the compound, the compound of Formula A for use in the treatments of the invention can comprise administering not more than six dosage amounts each comprising 300 mg of the compound, in a 24 hour period, wherein each dosage amount can be a tablet.

The dosage form can be a tablet comprising microcrystalline cellulose as a diluent, croscarmellose sodium as a disintegrant, polyvinyl pyrrolidone as a binder, and optionally magnesium stearate as a lubricant. In a preferred tablet, the compound of Formula A comprises: (i) at least about 40 wt% of the tablet (more specifically about 40 wt% to about 60 wt%), compared to the total mass of the tablet; (ii) about 25 wt% to about 60 wt% of the diluent (more specifically about 25 wt% to about 40 wt%, compared to the total mass of the tablet; (iii) about 1 wt% to about 15 wt% of the disintegrant (more specifically about 2 wt% to about 6 wt%), compared to the total mass of the tablet; (iv) about 1 wt% to about 20 wt% of the binder (more specifically about 2 wt% to about 5 wt%), compared to the total mass of the tablet; and when present, (v) about 0.1 to about 5 wt% lubricant (more specifically about 0.1 wt% to about 1.5 wt%), compared to the total mass of the tablet. The dosage form can be a tablet containing 300 mg of the compound.

The tablet can further comprise extragranular excipients comprising: microcrystalline cellulose as an extragranular diluent, croscarmellose sodium as an extragranular disintegrant, polyvinyl pyrrolidone as an extragranular binder, and/or magnesium stearate as an extragranular lubricant.

The dosage forms described herein (e.g. the tablets) can be film coated, wherein the film coating can comprise one or more of hypromellose, lactose monohydrate, titanium dioxide and triacetin.

### Further specifics of the compound of Formula A for use in the treatments of the invention

As shown herein, the compound of Formula A has a rapid onset of action. Specifically, the compound of Formula A is a potent inhibitor of plasma kallikrein activity and is highly effective at interrupting the contact activation system's positive feedback loop between plasma kallikrein, prekallikrein, Factor XII (FXII), and Factor XIIa (FXlla). The pharmacokinetic and pharmacodynamic data provided herein demonstrate that these effects are shown quickly after oral administration of the compound of Formula A. Accordingly, the compound of Formula A for use in the treatments of the invention are fast acting and are thus particularly suited to treating HAE on-demand.

As discussed above, the compound of Formula A for use in the treatments of the invention are particularly advantageous when the concentration of the compound of Formula A is at least 500 ng/mL in plasma. A plasma concentration of at least 500 ng/mL can be observed following administration of a dosage amount of at least about 60 mg (more specifically, at least about 70 mg or about 80 mg) of the compound of Formula A.

The compound of Formula A for use in the treatments according to the invention provide rapid protection from HK (high molecular weight kininogen) cleavage that are particularly suited to prophylactically reducing the chances of an acute HAE attack and/or to shorten the severity (or even halt) an ongoing acute HAE attack. As described here, the compound of Formula A for use in the treatments according to the invention also have a prolonged pharmacodynamic effect. The pharmacodynamic effects of the compound of Formula A that are related to treating HAE include providing protection from HK cleavage, which as discussed above, can cause an acute HAE attack. For example, the compound of Formula A can provide protection from HK cleavage by at least (i) inhibiting plasma kallikrein, (ii) reducing cleavage of plasma prekallikrein, and/or (iii) reducing the generation of Factor XIIa from Factor XII.

In some embodiments, the compound of Formula A for use in the treatments according to the invention can provide protection from HK (high molecular weight kininogen) cleavage within one hour post-dosage amount, and in particular when the dosage amount of the compound of Formula A is at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg). In some embodiments, the compound of Formula A for use in the treatments according to the invention can provide protection from HK (high molecular weight kininogen) cleavage within 45 minutes post- dosage amount, or within 30 minutes post- dosage amount. In these embodiments, protection from HK (high molecular weight kininogen) cleavage is determined by comparing HK levels in untreated plasma with HK levels in treated plasma *i.e*. plasma from subjects that have received a dosage amount of the compound of Formula A, and then activating the plasma with dextran sulfate to activate the contact system to induce HK cleavage. If the HK level in the treated plasma is above the HK level in the untreated plasma, then the HK has been protected from HK cleavage in the activated plasma.

In some embodiments of the invention, the treatment can inhibit at least 80% of plasma kallikrein activity within 30 minutes post-dosage amount, and in particular when the dosage amount of the compound of Formula A is at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg). In some embodiments of the invention, the treatment can inhibit at least 90% of plasma kallikrein activity within 30 minutes post-dosage amount, and in particular when the dosage amount of the compound of Formula A is at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg). In some embodiments of the invention, the treatment can inhibit at least 95% of plasma kallikrein activity within 30 minutes post-dosage amount, and in particular when the dosage amount of the compound of Formula A (or a pharmaceutically acceptable salt and/or solvate thereof is at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg). In embodiments where the inhibition of plasma kallikrein activity is mentioned, inhibition of plasma kallikrein activity is determined by time-dependent hydrolysis of fluorogenic substrate (e.g. (H-D-Pro-Phe-Arg-AFC; Peptide Protein Research) according to procedures known in the art. In these embodiments, inhibition of plasma kallikrein activity is determined in plasma obtained from subjects that have taken a dosage amount of the compound of Formula A which has subsequently been activated with dextran sulfate to emulate a HAE situation.

In some embodiments of the invention, a therapeutically effective concentration of the compound of Formula A can be achieved within 20 minutes post-dosage amount.

In some embodiments of the invention, the Tₘₐₓ of the compound of Formula A can be between 30 minutes and 3 hours post-dosage amount, preferably between 30 minutes and 2 hours post-dosage amount.

In some embodiments of the invention, the treatment can inhibit at least 90% of plasma kallikrein activity for at least the period of time between 45 minutes and 2 hours post-dosage amount, and in particular when the dosage amount of the compound of Formula A is between 100 mg and 200 mg (preferably 160 mg). In some embodiments, the treatment can inhibit at least 90% of plasma kallikrein activity for at least the period of time between 20 minutes and 4 hours post-dosage amount, and in particular when the dosage amount of the compound of Formula A is between 100 mg and 200 mg (preferably 160 mg). In some embodiments, the treatment can inhibit at least 90% of plasma kallikrein activity for at least the period of time between 30 minutes and 10 hours post-dosage amount, and in particular when the dosage amount of the compound of Formula A is between 300 mg and 800 mg (preferably 600 mg). In some embodiments, the treatment can inhibit at least 95% of plasma kallikrein activity for at least the period of time between 20 minutes and 6 hours post-dosage amount, and in particular when the dosage amount of the compound of Formula A is between 300 mg and 800 mg (preferably 600 mg). In some embodiments, the treatment can inhibit at least 99% of plasma kallikrein activity for at least the period of time between 20 minutes and 6 hours post-dosage amount, and in particular when the dosage amount of the compound of Formula A is between 300 mg and 800 mg (preferably 600 mg). Again, in these embodiments, inhibition of plasma kallikrein activity is determined in plasma obtained from subjects that have taken a dosage amount of the compound of Formula A which has subsequently been activated with dextran sulfate to emulate a HAE situation.

In some embodiments, the pharmacodynamic effects of the compound of Formula A that are related to treating HAE can be maintained for at least 12 hours post-dosage amount, and in particular when the dosage amount of the compound of Formula A is between 300 mg and 800 mg (preferably 600 mg). In some embodiments, the treatment can inhibit at least 50% of plasma kallikrein activity for at least 10 hours post-dosage amount, and in particular when the dosage amount of the compound of Formula A is between 100 mg and 200 mg (preferably 160 mg). In these embodiments, pharmacodynamic effects means at least (i) inhibition of plasma kallikrein, (ii) protection from HK cleavage / reduction of HK cleavage, (iii) protection from (or a reduction of) Factor XII cleavage to generate Factor XIIa, and/or (iv) protection from (or a reduction of) plasma prekallikrein cleavage to generate plasma kallikrein. The compound of Formula A for use in treatments according to the invention are therefore suitable candidates for being advantageously efficacious treatments of acute HAE attacks because they are fast-acting and potent (e.g. inhibitory) over a sufficiently long period of time.

As discussed above, in any of the treatments of the invention, the compound of Formula A can inhibit plasma kallikrein.

In any of the treatments of the invention, particularly following a dosage amount of the compound of Formula A of at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg), the compound of Formula A can inhibit Factor XII cleavage to generate Factor XIIa. In any of the treatments of the invention, particularly following a dosage amount of the compound of Formula A of at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg), the compound of Formula A can inhibit plasma prekallikrein cleavage into plasma kallikrein. In any of the treatments of the invention, particularly following a dosage amount of the compound of Formula A of at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg), the compound of Formula A can result in the inhibition (e.g. blockage) of contact system activation for up to 6 hours post-dosage amount. In some embodiments, where a dosage amount of at least about 60 mg (more specifically, at least about 70 mg or about 80 mg such as about 80 mg to about 900 mg, about 100 mg to about 800 mg, about 200 mg to about 700 mg, about 300 mg to about 600 mg, or about 400 mg to about 600 mg, specifically 600 mg) is administered, the contact system activation can be inhibited (e.g. blocked) for at least 6 hours e.g. for between 6 hours and 12 or 18 hours post-dosage amount.

### Figures

In the Figures, the term "Compound" means the compound of Formula A.
Figure 1: X-ray powder diffraction pattern of the compound of Formula A as generated in Example 1.
Figure 2A: Assay results showing plasma kallikrein inhibition activity of the compound of Formula A and a C1 inhibitor C1-INH in dextran sulfate (DXS)-activated diluted plasma.
Figure 2B: Assay results showing plasma kallikrein inhibition activity of the compound of Formula A and a C1 inhibitor (C1-INH) in DXS-activated undiluted plasma.
Figure 3A: Assay results comparing the plasma kallikrein inhibition activity of the compound of Formula A and C1-INH in DXS-activated diluted plasma.
Figure 3B: Assay results comparing inhibition activity of the compound of Formula A and C1-INH following addition to pre-activated undiluted human plasma. Data are expressed as total fluorescence over time (Fluorescence Units) mean ± SEM of n=3 experiments.
Figure 4A: Assay (bioanalytical) results showing plasma concentrations of the compound of Formula A between 0 and 24 hours post-dose, in fasted subjects from eight (8) single ascending dose cohorts.
Figure 4B: Table of Cₘₐₓ values determined from the assay (bioanalytical) results shown in Figure 4A.
Figure 5A: Assay results showing plasma kallikrein activity in DXS-activated undiluted plasma for cohorts 6 to 8 (160 mg, 300 mg, and 600 mg).
Figure 5B: Assay results showing the mean plasma kallikrein activity and mean plasma concentration of the compound of Formula A in undiluted plasma in subjects from cohort 8 (600 mg dose).
Figure 6A: Assay results showing the mean fluorescent kinetic measurements indicating a lag time in catalytic activity during contact system activation in DXS-activated undiluted plasma of a subject who has received a 600 mg dose of the compound of Formula A.
Figure 6B: An enlargement of Figure 6A between 0 and 5 mins following catalytic activation.
Figure 7: Assay results showing mean percent HK protection at selected time points post-dosage in DXS-activated undiluted plasma for cohorts 6 to 8 (160 mg, 300 mg, and 600 mg), and a representation WES gel image of the immunoblot data.
Figure 8: Assay results showing the effect of the compound of Formula A on DXS-activated HK cleavage at selected time points post-dosage in cohort 8 (600mg), and a representation WES gel image of the immunoblot data.
Figure 9: Assay results showing the effect of the compound of Formula A on DXS-activated plasma prekallikrein (PPK) cleavage, at selected time points post-dosage in cohort 8 (600mg), and a representation WES gel image of the immunoblot data.
Figure 10: Assay results showing the effect of the compound of Formula A on DXS- activated generation of FXlla, at selected time points post-dosage in cohort 8 (600mg), and a representation WES gel image of the immunoblot data.
Figure 11: Assay (bioanalytical) results showing the effect of the plasma concentration of the compound of Formula A at various stages post-dose in cohort 8 (600mg) at time points selected for HK, FXlla, PPK analysis.
Figure 12: Assay results showing no significant food effect on the plasma kallikrein inhibitory activity of the compound of Formula A in DXS-activated undiluted plasma.
Figures 13A and 13B: Assay results showing a time course of dextran sulfate-activated cleavage of HK in HAE whole undiluted plasma determined using western blotting, and a representative blot image.
Figures 14A and 14B: Assay results showing the dose response of the compound of Formula A on full length HK levels in dextran sulfate-activated healthy control plasma and HAE plasma, and representative WES system gel images.
Figure 15: Preliminary pharmacokinetic data from the currently ongoing phase 2 study.
Figure 16A: Mean plasma concentrations over time of 4 cohorts in the phase 1 multiple dose study.
Figure 16B: Mean plasma concentrations over time (semi-logarithmic scale) of 4 cohorts in the phase 1 multiple dose study.

Embodiments provided herein may be more fully understood by reference to the following examples. These examples are meant to be illustrative of treatments provided herein, but are not in any way limiting. Indeed, the scope of the invention is defined by the claims.

While examples of certain particular embodiments are provided herein, it will be apparent to those skilled in the art that various changes and modifications may be made. Such modifications are also intended to fall within the scope of the appended claims.

### General Experimental Details

In the following examples, the following abbreviations and definitions are used:

| | |
|---|---|
| Aq | Aqueous solution |
| DCM | Dichloromethane |
| DMF | N,N-Dimethylformamide |
| DMSO | Dimethyl sulfoxide |
| DXS | Dextran sulfate |
| EtOAc | Ethyl Acetate |
| FXII | Factor XII |
| FXlla | Factor XIIa |
| HK | High molecular weight kininogen |
| Hrs | Hours |
| HOBt | Hydroxybenzotriazole |
| IPA | 2-Propanol / Propan-2-ol / Iso-propanol |
| LCMS | Liquid chromatography mass spectrometry |
| Me | Methyl |
| MeCN | Acetonitrile |
| MeOH | Methanol |
| Min | Minutes |
| MS | Mass spectrum |
| NMR | Nuclear magnetic resonance spectrum - NMR spectra were recorded at a frequency of 400MHz unless otherwise indicated |
| Pet. Ether | Petroleum ether fraction boiling at 60-80°C |
| Ph | Phenyl |
| PKa | Plasma kallikrein |
| PPK | Plasma prekallikrein |
| SWFI | Sterile water for injection |
| Rt | room temperature |
| THF | Tetrahydrofuran |
| XRPD | X-ray powder diffraction |

All reactions were carried out under an atmosphere of nitrogen unless specified otherwise.

¹H NMR spectra were recorded on a Bruker (400MHz) or on a JEOL (400MHz) spectrometer with reference to deuterium solvent and at rt.

Molecular ions were obtained using LCMS which was carried out using a Chromolith Speedrod RP-18e column, 50 × 4.6 mm, with a linear gradient 10% to 90% 0.1% HCO₂H/MeCN into 0.1% HCO₂H/H₂O over 13 min, flow rate 1.5 mL/min, or using Agilent, X-Select, acidic, 5-95% MeCN/water over 4 min. Data was collected using a Thermofinnigan Surveyor MSQ mass spectrometer with electospray ionisation in conjunction with a Thermofinnigan Surveyor LC system.

Alternatively, molecular ions were obtained using LCMS which was carried out using an Agilent Poroshell 120 EC-C18 (2.7µm, 3.0 × 50mm) column with 0.1% v/v Formic acid in water [eluent A]; MeCN [eluent B]; Flow rate 0.8mL/min and 1.5 minutes equilibration time between samples, gradient shown below. Mass detection was afforded with API 2000 mass spectrometer (electrospray).

### Gradient:

| **Time (min)** | **Eluent A (%)** | **Eluent B (%)** |
|---|---|---|
| 0.00 | 95 | 5 |
| 0.20 | 95 | 5 |
| 2.00 | 5 | 95 |
| 3.00 | 5 | 95 |
| 3.25 | 95 | 5 |
| 3.50 | 95 | 5 |

Where products were purified by flash chromatography, 'silica' refers to silica gel for chromatography, 0.035 to 0.070 mm (220 to 440 mesh) (e.g. Merck silica gel 60), and an applied pressure of nitrogen up to 10 p.s.i accelerated column elution. Reverse phase preparative HPLC purifications were carried out using a Waters 2525 binary gradient pumping system at flow rates of typically 20 mL/min using a Waters 2996 photodiode array detector.

All solvents and commercial reagents were used as received.

Chemical names were generated using automated software such as the Autonom software provided as part of the ISIS Draw package from MDL Information Systems or the Chemaxon software provided as a component of MarvinSketch or as a component of the IDBS E-WorkBook.

X-Ray Powder Diffraction patterns were collected on a Philips X-Pert MPD diffractometer and analysed using the following experimental conditions (Method A), unless otherwise specified:
Tube anode: Cu
Generator tension: 40 kV
Tube current: 40 mA
Wavelength alpha1: 1.5406 Å
Wavelength alpha2: 1.5444 Å
Start angle [2θ]: 4
End angle [2θ]: 40
Continuous scan

Approximately 2 mg of sample under analysis was gently compressed on the XRPD zero back ground single obliquely cut silica sample holder. The sample was then loaded into the diffractometer for analysis.

### Example 1 - Preparation of the compound of Formula A

### A. 1-(4-Hydroxymethyl-benzyl)-1H-pyridin-2-one

4-(Chloromethyl)benzylalcohol (5.0 g, 31.93 mmol) was dissolved in acetone (150 mL). 2-hydroxypyridine (3.64 g, 38.3 mmol) and potassium carbonate (13.24 g, 95.78 mmol) were added and the reaction mixture was stirred at 50 °C for 3 hrs after which time the solvent was removed *in vacuo* and the residue taken up in chloroform (100 mL). This solution was washed with water (30 mL), brine (30 mL), dried (Na₂SO₄) and evaporated *in vacuo.* The residue was purified by flash chromatography (silica), eluent 3% MeOH / 97% CHCl₃, to give a white solid identified as 1-(4-hydroxymethyl-benzyl)-1H-pyridin-2-one (5.30g, 24.62mmol, 77% yield).
[M+Na]⁺ = 238

### B. 1-(4-Chloromethyl-benzyl)-1H-pyridin-2-one

1-(4-Hydroxymethyl-benzyl)-1H-pyridin-2-one (8.45 g, 39.3 mmol), dry DCM (80 mL) and triethylamine (7.66 ml, 55.0 mmol) were cooled in an ice bath. Methanesulfonyl chloride (3.95 ml, 51.0 mmol) was added and stirred in ice bath for 15 min. The ice bath was removed and stirring continued at rt temperature overnight. The reaction mixture was partitioned between DCM (100 mL) and saturated aqueous NH₄Cl solution (100 mL). The aqueous layer was extracted with further DCM (2 × 50 mL) and the combined organics washed with brine (50 mL), dried over Na₂SO₄, filtered and concentrated to give 1-(4-chloromethyl-benzyl)-1H-pyridin-2-one (8.65 g, 36.6 mmol, 93 % yield) as a pale yellow solid.
[MH]⁺ = 234.1

### C. Methyl 3-(methoxymethyl)-1-(4-((2-oxopyridin-1(2H)-yl)methyl)benzyl)-1H-pyrazole-4-carboxylate

Potassium carbonate (519 mg, 3.76 mmol) was added to a solution of methyl 3-(methoxymethyl)-1H-pyrazole-4-carboxylate (320 mg, 1.88 mmol; CAS no. 318496-66-1 (synthesised according to the method described in WO 2012/009009)) and 1-(4-(chloromethyl)benzyl)pyridin-2(1H)-one (527 mg, 2.26 mmol) in DMF (5 mL) and heated at 60 °C overnight. The reaction mixture was diluted with EtOAc (50 mL) and washed with brine (2 × 100 mL), dried over magnesium sulfate, filtered and reduced *in vacuo.* The crude product was purified by flash chromatography (40 g column, 0-100% EtOAc in isohexanes) to afford two regioisomers. The second isomer off the column was collected to afford methyl 3-(methoxymethyl)-1-(4-((2-oxopyridin-1(2H)-yl)methyl)benzyl)-1H-pyrazole-4-carboxylate (378 mg, 1.01 mmol, 53.7 % yield) as a colourless gum.
[MH]⁺ = 368.2

### D. 3-(Methoxymethyl)-1-(4-((2-oxopyridin-1(2H)-yl)methyl)benzyl)-1H-pyrazole-4-carboxylic acid

To methyl 3-(methoxymethyl)-1-(4-((2-oxopyridin-1(2H)-yl)methyl)benzyl)-1H-pyrazole-4-carboxylate (3.77 g, 10.26 mmol) in THF (5 mL) and MeOH (5 mL) was added 2M NaOH solution (15.39 ml, 30.8 mmol) and stirred at rt overnight. 1M HCl (50 mL) was added and extracted with EtOAc (50 mL). The organic layer was washed with brine (50 mL), dried over magnesium sulfate, filtered and reduced *in vacuo* to give 3-(methoxymethyl)-1-(4-((2-oxopyridin-1(2H)-yl)methyl)benzyl)-1H-pyrazole-4-carboxylic acid (1.22 g, 3.45 mmol, 33.6 % yield) as a white powder.
[MH]⁺ = 354.2

### E. 3-Fluoro-4-methoxy-pyridine-2-carbonitrile

To a large microwave vial, copper (I) cyanide (1.304 g, 14.56 mmol) was added to a solution of 2-bromo-3-fluoro-4-methoxypyridine (1 g, 4.85 mmol) in DMF (5 mL). The reaction vial was sealed and heated to 100 °C for 16 hrs. The reaction mixture was diluted with water (20 mL) and EtOAc (20 mL). The thick suspension was sonicated and required additional water (40 mL) and EtOAc (2 × 50 mL) with sonication to break-up the solid precipitated. The combined layers were filtered through a plug of celite and the organic layer isolated, washed with brine (50 mL), dried over magnesium sulfate, filtered and the solvent removed under reduced pressure to give a pale green solid identified as the desired compound 3-fluoro-4-methoxy-pyridine-2-carbonitrile (100 mg, 0.578 mmol, 12 % yield)

### F. (3-Fluoro-4-methoxy-pyridin-2-ylmethyl)-carbamic acid tert-butyl ester

3-Fluoro-4-methoxy-pyridine-2-carbonitrile (100 mg, 0.578 mmol) was dissolved in anhydrous methanol (10 mL, 247 mmol) and nickel chloride hexahydrate (14 mg, 0.058 mmol) was added followed by di-tert-butyl dicarbonate (255 mg, 1.157 mmol). The resulting pale green solution was cooled in an ice-salt bath to -5 °C and then sodium borohydride (153 mg, 4.05 mmol) was added portionwise maintaining the reaction temperature ^{~}0 °C. The deep brown solution was left to stir at 0 °C and slowly allowed to warm to rt and then left to stir at rt for 3 hrs. The reaction mixture was evaporated to dryness at 40°C to afford a black residue which was diluted with DCM (10 mL) and washed with sodium hydrogen carbonate (10 mL). An emulsion formed so the organics were separated *via* a phase separating cartridge and concentrated. The crude liquid was purified by chromatography eluting with EtOAc / iso-Hexane to afford the title compound, (3-fluoro-4-methoxy-pyridin-2-ylmethyl)-carbamic acid tert-butyl ester as a clear yellow oil (108 mg, 62 % yield)
[MH]⁺ = 257

### G. C-(3-Fluoro-4-methoxy-pyridin-2-yl)-methylamine hydrochloride salt

(3-Fluoro-4-methoxy-pyridin-2-ylmethyl)-carbamic acid tert-butyl ester (108mg, 0.358mmol) was taken up in iso-propyl alcohol (1 mL) and then HCl (6N in iso-propyl alcohol) (1 mL, 0.578 mmol) was added at rt and left to stir at 40°C for 2 hrs. The reaction mixture was concentrated under reduced pressure and then triturated with ether, sonicated and then decanted to give a cream coloured solid (75 mg, 55% yield) identified as C-(3-fluoro-4-methoxy-pyridin-2-yl)-methylamine hydrochloride salt.
[MH]⁺ = 157

### Example 1a - N-[(3-Fluoro-4-methoxypyridin-2-yl)methyl]-3-(methoxymethyl)-1-({4-[(2-oxopyridin-1-vl)methyl]phenyl}methyl)pyrazole-4-carboxamide (Compound of Formula A)

3-(Methoxymethyl)-1-(4-((2-oxopyridin-1(2H)-yl)methyl)benzyl)-1H-pyrazole-4-carboxylic acid (825 mg, 2.34 mmol) and C-(3-fluoro-4-methoxy-pyridin-2-yl)-methylamine hydrochloride salt (450 mg, 2.34 mmol) were dissolved in DCM while cooling to 0°C. 1-Ethyl-3-(3-dimethylaminopropyl)carbodiimide hydrochloride (627.0 mg, 3.27 mmol), HOBt (378.8 mg, 2.80 mmol) and triethylamine (1.63 mL, 1182 mmol) were added while stirring, the mixture allowed to warm to rt and stirring continued for 20 hrs. Chloroform (50 mL) was added, the mixture was washed with saturated NaHCO₃(aq) and reduced *in vacuo.* The crude material was purified by chromatography eluting with methanol/DCM. The solvent was removed *in vacuo* and the resulting solid triturated with diethyl ether. The resulting solids were collected by filtration to afford the compound of Formula A.
[MH]⁺ = 492.0

NMR (CD₃OD) δ: 3.41 (3H, s), 4.03 (3H, s), 4.65 (2H, s), 4.72 (2H, d, J=2.3Hz), 5.24 (2H, s), 5.37 (2H, s), 6.44 (1H, td, J = 1.4, 6.8Hz), 6.62 (1H, d, J = 9.0Hz), 7.18-7.22 (1H, m), 7.31-7.38 (4H, m), 7.56-7.60 (1H, m), 7.75 (1H, dd, J = 1.9, 7.1Hz), 8.18 (1H, s), 8.27 (1H, d, J = 5.6Hz) ppm.

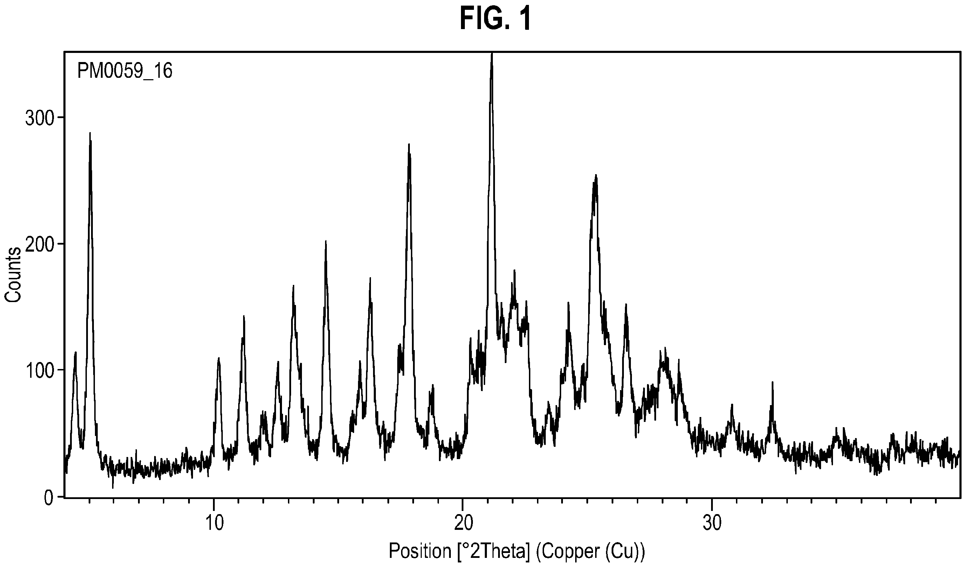

An XRPD diffractogram of the compound of Formula A resultant from the above procedure is shown in Figure 1.

Peak position table:

| **No.** | **Pos. [°2Th.]** | **Rel. Int. [%]** |
|---|---|---|
| | | |
| 1 | 4.436 | 32.36 |
| 2 | 5.0471 | 58.74 |
| 3 | 10.2255 | 43.07 |
| 4 | 11.2061 | 48.44 |
| 5 | 12.0101 | 16.4 |
| 6 | 12.5494 | 37.17 |
| 7 | 13.165 | 67.26 |
| 8 | 14.4984 | 38.94 |
| 9 | 15.8919 | 23.54 |
| 10 | 16.2983 | 34.56 |
| 11 | 17.4492 | 36.63 |
| 12 | 17.8564 | 71.49 |
| 13 | 18.6888 | 21.9 |
| 14 | 20.285 | 26.12 |
| 15 | 21.1598 | 100 |
| 16 | 22.04 | 87.76 |
| 17 | 22.5857 | 36.38 |
| 18 | 23.4408 | 14.33 |
| 19 | 24.3045 | 31.11 |
| 20 | 25.1655 | 78.97 |
| 21 | 25.3728 | 93.91 |
| 22 | 26.4946 | 56.79 |
| 23 | 27.991 | 76.91 |
| 24 | 28.7495 | 22.99 |
| 25 | 30.7611 | 13.4 |
| 26 | 32.413 | 17.2 |
| 27 | 37.2144 | 14.13 |
| 28 | 38.1171 | 14.14 |

### Example 2 - Preparation of a dosage form comprising the compound of Formula A

### Blending and Roller Compaction

Equipment: Freund Vector TFC Lab Micro Roller Compactor and Granulator *(the roller compactor and granulator are separate entities).* The equipment parameters are below:

| **Parameter** | **Range Used** |
|---|---|
| Screw Speed (rpm) | 10.0 - 20.0 |
| Roll Speed (rpm) | 1.0 - 2.0 |
| Roll Force (kN) | 0.50 - 12.00 |
| Granulator Screen Size (mm) | 1 |

### Method

Two tablet formulations (Tablets A and B) were prepared according to the following method at 30g blend scale to produce tablets having components in the amounts shown below.

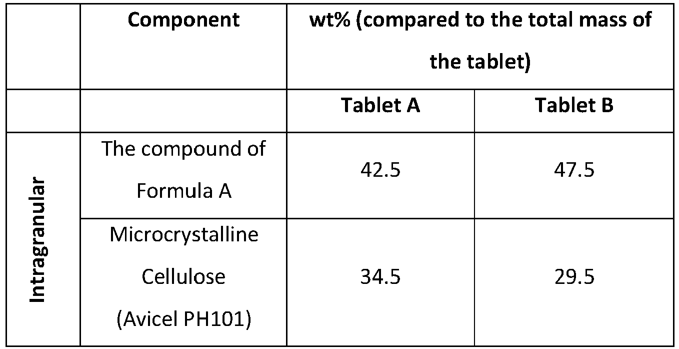

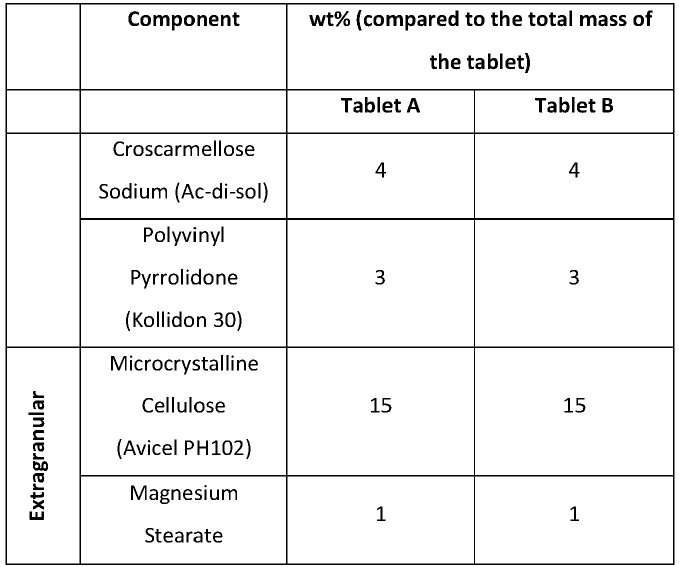

For each of the tablets, blends were prepared by passing the intragranular components through a 355 µm sieve at a suitable scale for the scope of the roller compactor in a glass vessel using a Turbula Blender at 34 rpm. The blend was then run through the roller compactor using the parameters described above. The ribbons produced were collected into a suitably sized container. The collected ribbons were then subjected to the granulator fixed with a 1 mm screen and the resultant granules were collected for further downstream processing.

### Tabletting

Equipment: RIVA Mini single station Tablet Press. The equipment parameters are shown below:

| **Parameter** | **Range Used** |
|---|---|
| Tooling Diameter | 8 mm round |
| Force (kN) | 6 - 10 |
| Fill Weight (mg) | 178 - 300 |

The granules were subsequently blended with their extragranular excipients, respectively. The extragranular excipients were prepared by screening through a 355 µm sieve in a glass vessel using a Turbula Blender at 34 rpm. The target tablet weight was then dispensed and manually compressed into tablets. Tablet A was compressed at 7.2 to 8.8 kN compression force. Tablet B was compressed at 6.9 to 7.7 kN compression force.

The tablets were found to be robust. Tablets A and B were subsequently submitted for long-term stability testing.

The production of tablets per the method described above has been scaled to 180g with a roller compaction time of approximately 60 minutes.

### Example 3 - Comparison of the compound of Formula A with a C1 inhibitor (C1-INH)

Aim: To identify the biochemical and biophysical properties of the compound of Formula A that contribute to its optimal efficacy in controlling the Kallikrein Kinin System in plasma. These properties are then compared to C1-INH as a therapeutic benchmark for HAE.

### Methods:

Plasma kallikrein inhibitory activity *in vitro* was determined using standard published methods (see e.g. Johansen et al., Int. J. Tiss. Reac. 1986, 8, 185; Shori et al., Biochem. Pharmacol., 1992, 43, 1209; Stürzebecher et al., Biol. Chem. Hoppe-Seyler, 1992, 373, 1025). Human plasma kallikrein (Protogen) was incubated at 25 °C with the fluorogenic substrate H-DPro-Phe-Arg-AFC and various concentrations of the test compound. Residual enzyme activity (initial rate of reaction) was determined by measuring the change in optical absorbance at 410nm and the IC₅₀ value for the test compound was determined.

The rate of formation of the enzyme-inhibitor complex (Kₒₙ) was determined using purified PKa rapidly mixed with a solution containing fluorogenic substrate and a concentration range of inhibitor. The time-dependent establishment of inhibition was then used to calculate the rate of formation of the enzyme-inhibitor complex for each concentration of inhibitor. The Kₒₙ was calculated by plotting the rate of inhibition versus the inhibitor concentration. Data in Table 1 are presented in µM⁻¹ sec⁻¹.

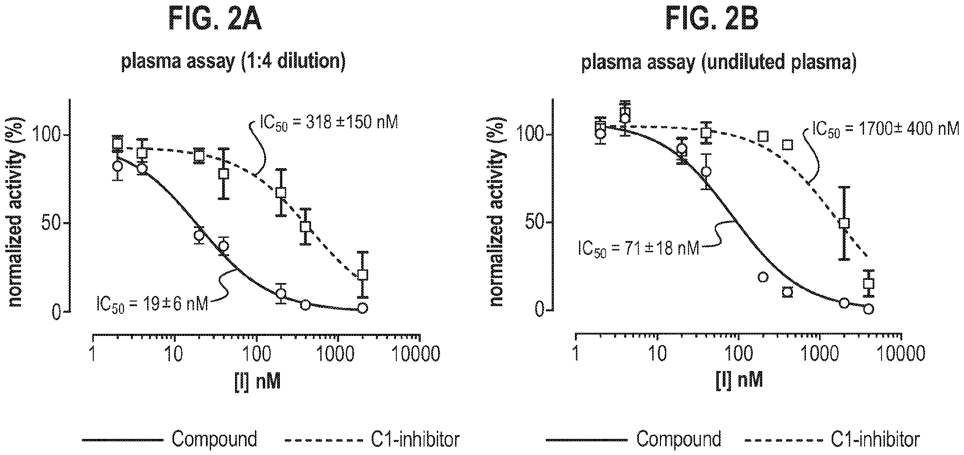

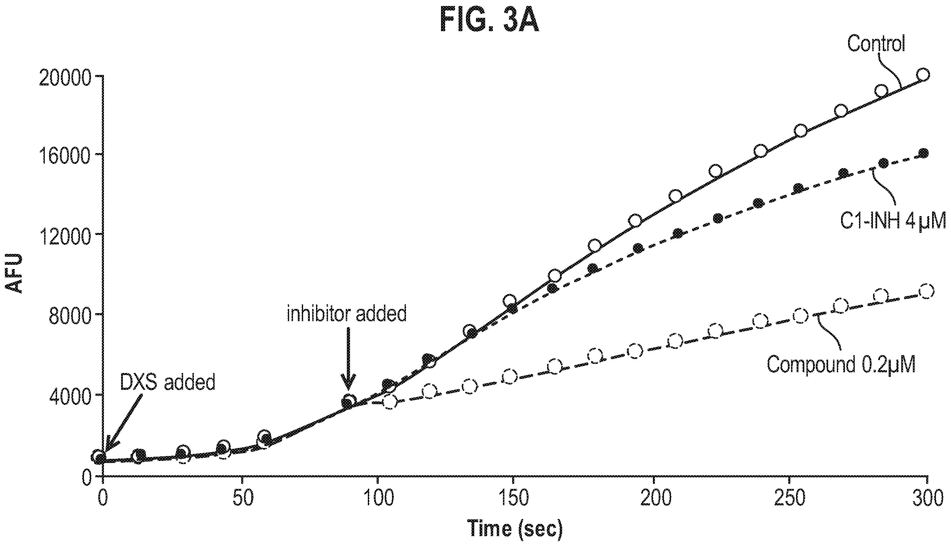

Catalytic activity of PKa in dextran sulfate-activated (DXS, Sigma; 10 µg/ml) plasma (1:4 diluted or undiluted, VisuCon-F control plasma, Affinity Biologicals Inc) was determined by the time-dependent hydrolysis of fluorogenic substrate. For IC₅₀ and efficacy determination, the compound of Formula A or C1-INH (Sigma Cat #E0518) were added either before (Figures 2A and 2B) or after (Figure 3A) the addition of DXS to the plasma.

DXS-activated cleavage of HK in undiluted plasma was performed in the absence or presence of 300 nM PKa inhibitor and quantified by SDS-PAGE gel electrophoresis, using 7.5% Criterion TGX Precast gels (Biorad). Transfer was made onto Immunobilon-FL PVDF membrane. Image analysis was done using the LICOR imaging system. Mouse monoclonal anti-HK antibody (MAB15692, R&D systems) was used for traditional immunoblotting. Data presented as % of HK remaining after 20 min incubation with DXS compared to HK levels in unactivated plasma (Table 1).

Plasma free fraction was determined using "Rapid Equilibrium Dialysis" system (Thermo Scientific), test compounds were prepared at 5 µM in neat human plasma and dialysed against phosphate buffer for 5 hrs at 37°C. Quantification of the compound partitioned in two chambers of the dialysis device was performed via LCMS/ MS. Fraction of compound unbound to plasma proteins presented as % of total.

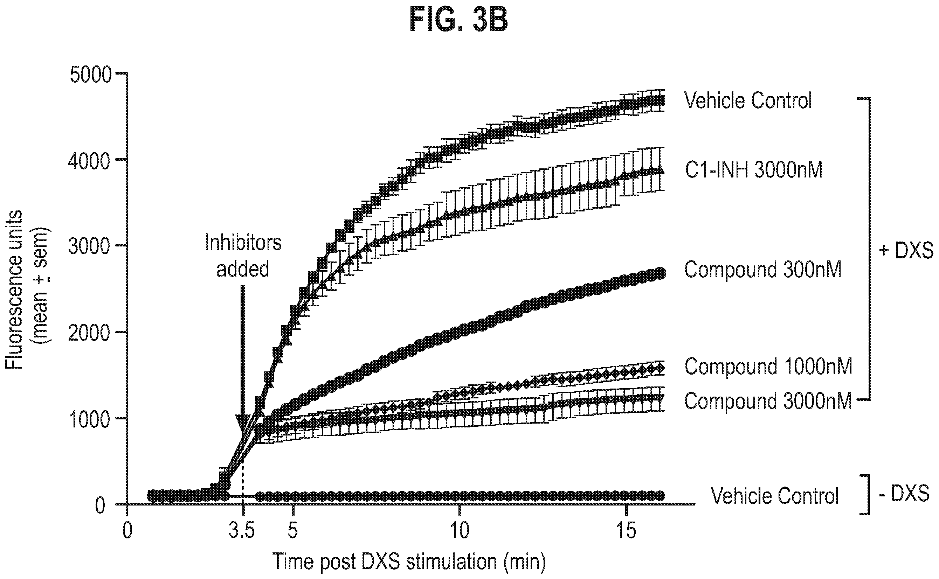

The ability of the compound to inhibit the enzyme activity of pre-activated plasma was assessed by addition of the compound after DXS stimulation. Aliquots of plasma (20 µL) were mixed with a 2.5 µL solution containing 1,300 mM fluorogenic substrate (H-DPro-Phe-Arg-AFC) and a 2.5 µL solution of dextran sulphate (DXS; 100 µg/mL) which acted as an activator of the plasma kallikrein-kinin pathway. Enzyme activity was immediately measured by monitoring the accumulation of fluorescence liberated from the substrate by substrate cleavage over 16 minutes. At 3.5 minutes post DXS addition 5 µl of inhibitors or water control are were added into each well. The compound was tested at concentrations of 300, 1000 and 3000 nM. C1-INH at a concentration of 3000 nM and vehicle controls were also included. Data are presented in Figure 3B.

### Results:

As shown in Figure 2, in assays using the fluorogenic substrate, the compound of Formula A appears to be a highly potent inhibitor of PKa with 17-fold and 20-fold potency vs. exogenously added C1-INH in diluted plasma (Figure 2A) and undiluted plasma (Figure 2B), respectively.

**Table 1 showing the biochemical profile of the therapies tested in this example.**

| | **IC₅₀ purified enzyme nM** | **IC₅₀ plasma enzyme nM** | **Kₒₙ (µM⁻¹ sec⁻¹)** | **Plasma Free Fraction %** | **HK protection %** |
|---|---|---|---|---|---|
| C1-INH | 50 | 1700 | 0.04 | | |
| Compound of Formula A | 6 | 71 | 11 | 25 | 85 |

Figure 3A shows a comparison of the effects of the two inhibitors: compound of Formula A and C1-INH, on plasma kallikrein activity in plasma (diluted 1:4) activated with DXS. Both inhibitors were added at concentrations ten times their IC₅₀ to plasma approximately 100 seconds after the addition of the DXS.

Figure 3B shows that addition of the compound of Formula A after the activation of plasma causes rapid and dose dependent inhibition of enzyme activity compared to the slower action of C1-INH.

**Table 2 shows the biochemical potency and selectivity of the compound of Formula A against human isolated enzymes using literature methods as for the above-described in vitro plasma kallikrein assay.**

| **The compound** | **Ki (nM)** |
|---|---|
| Plasma Kallikrein | 3.02 |

| **Selectivity vs PKa** | **Fold** |
|---|---|
| Tissue Kallikrein (KLK1) | >6000 |
| Factor XIa | >6000 |
| Factor XIIa | >6000 |
| Thrombin | >6000 |
| Trypsin | >6000 |

### Example 4 - Phase I single ascending dose study in healthy males and food effect of the compound

Aim: To evaluate the pharmacodynamic (PD) effects of the compound of Formula A when orally administered using *ex vivo* whole plasma assays for plasma kallikrein catalytic activity and HK cleavage, in samples from a Phase 1 Single Ascending Dose Study in healthy adult males. Also, an aim was to investigate safety, tolerability and pharmacokinetic (PK) effects of the compound of Formula A when orally administered.

### Methods:

This study was a randomized, double-blind, placebo-controlled single ascending dose (SAD) and crossover studies for food effect and capsule/tablet formulations.

64 healthy male participants (n=6 active, 2 placebo per cohort, 8 SAD cohorts) were administered single ascending doses of the compound of Formula A: 5, 10, 20, 40, 80, 160, 300 or 600 mg in a capsule.

8 participants were administered 100 mg the compound of Formula A in a crossover study of the capsule and a tablet formulation.

12 participants were administered 600 mg the compound of Formula A in a food effect crossover study.

Samples for pharmacokinetic (PK) and PD assessment were taken at repeated intervals over 48 hours.

Plasma samples used for PK assessment were analysed using a validated liquid chromatography tandem mass spectrometry (LC MS/MS) method.

PD measurements were determined in dextran sulfate (DXS) stimulated undiluted plasma using a fluorogenic enzyme assay and capillary based HK cleavage immunoassay.

Catalytic activity of PKa in DXS-stimulated (Sigma; 10 µg/mL) plasma samples from the compound of Formula A phase 1 study was determined by the time-dependent hydrolysis of fluorogenic substrate in all samples from all parts of the study.

The time until appearance of detectable amidolytic enzyme activity in DXS-stimulated plasma (lag time) was calculated from the catalytic activity assay. The detection sensitivity of the rate of catalytic activity in plasma based on using a Spark (Tecan) fluorimeter is a fluorescence increase to reach 1ΔF unit/sec.

DXS-stimulated cleavage of HK, in undiluted plasma was quantified by capillary-based immunoassay on the Wes System (ProteinSimple) using monoclonal anti-HK antibody and chemiluminescence-based detection. Plasma kallikrein mediated HK cleavage in undiluted citrated human plasma was induced by contact system activation with DXS (6.25 µg/ml) at 4°C in selected samples from the SAD phase.

DXS-stimulated cleavage of plasma prekallikrein and Factor XII (FXII) were quantified by capillary-based immunoassay on the Wes System (ProteinSimple) analogously.

### Results:

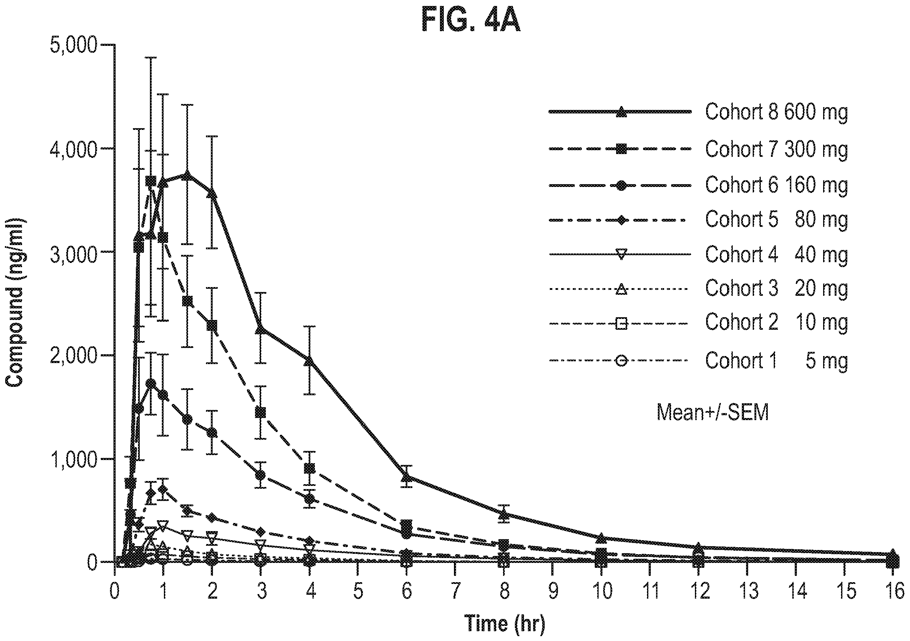

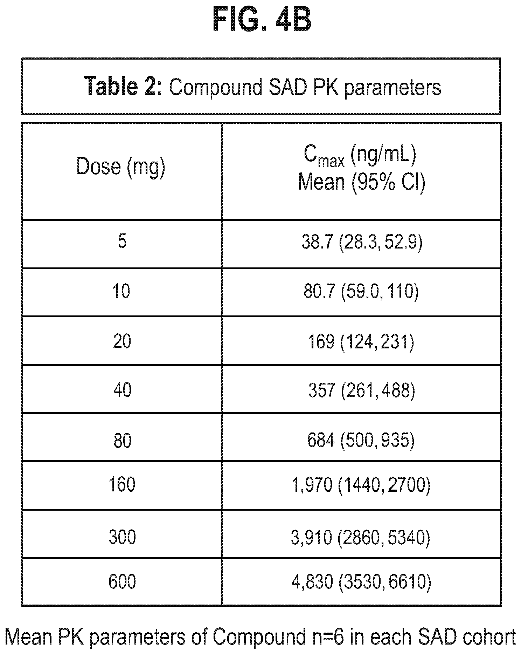

Figure 4A shows the plasma concentrations of the compound of Formula A from 0 to 24 hours post-dose. As can be seen, when orally administered, the compound of Formula A achieved rapid and dose-dependent plasma exposure over the range of doses tested from 5 mg to 600 mg. Figure 4A shows the concentration curves and Figure 4B shows the Cₘₐₓ for each SAD cohort. The compound of Formula A was administered as a capsule formulation and the subject was in the fasted state.

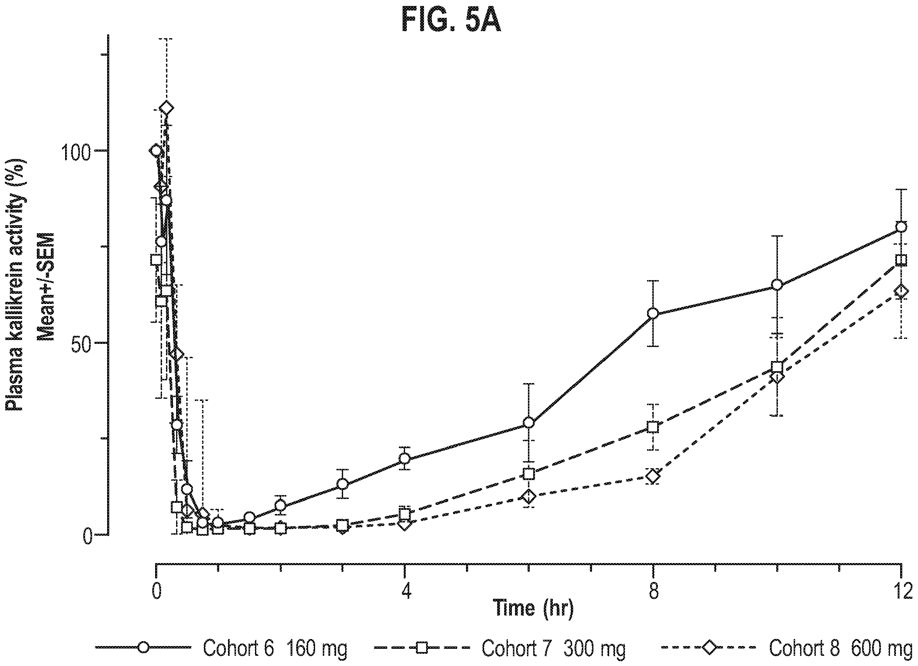

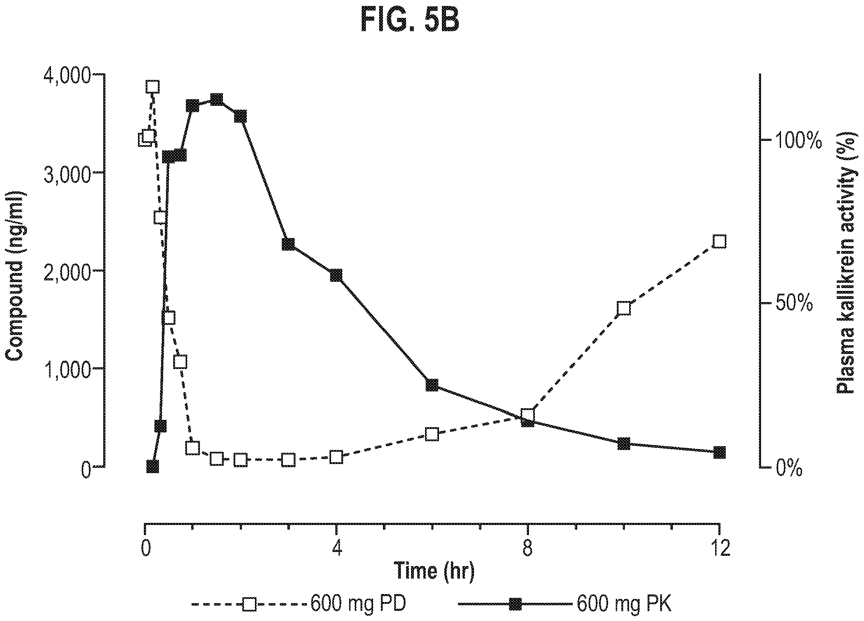

Figure 5A shows enzyme assays in activated undiluted plasma performed on samples from cohorts 6, 7, and 8. Doses 160 mg and above demonstrated >90% average inhibition of plasma kallikrein catalytic activity between 45 min and 2 hr for cohort 6, between 20 min and 4 hr for cohort 7. A 600 mg dose (cohort 8) provided >90% inhibition of plasma kallikrein catalytic activity between 30 min and 6 hr post-dose and >50% inhibition for 10hr (Figure 5B).

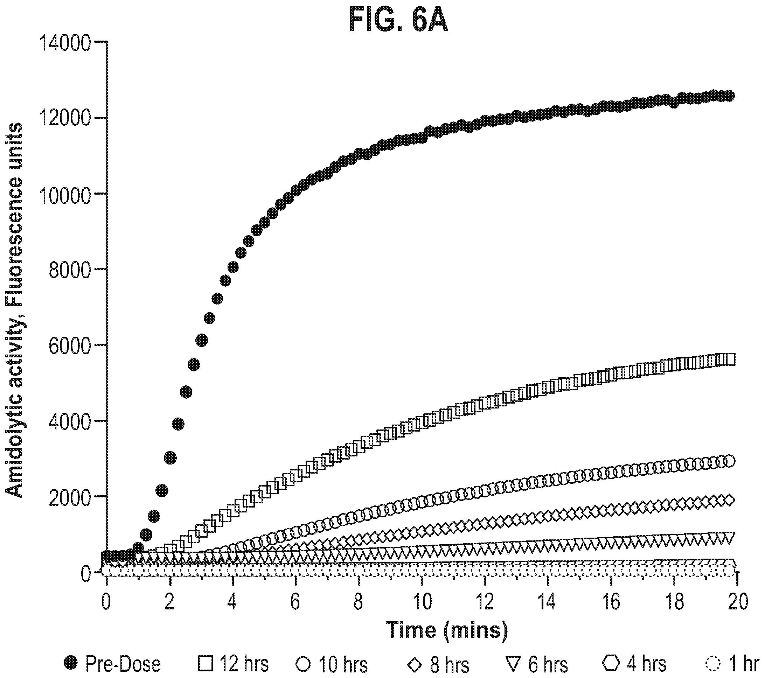

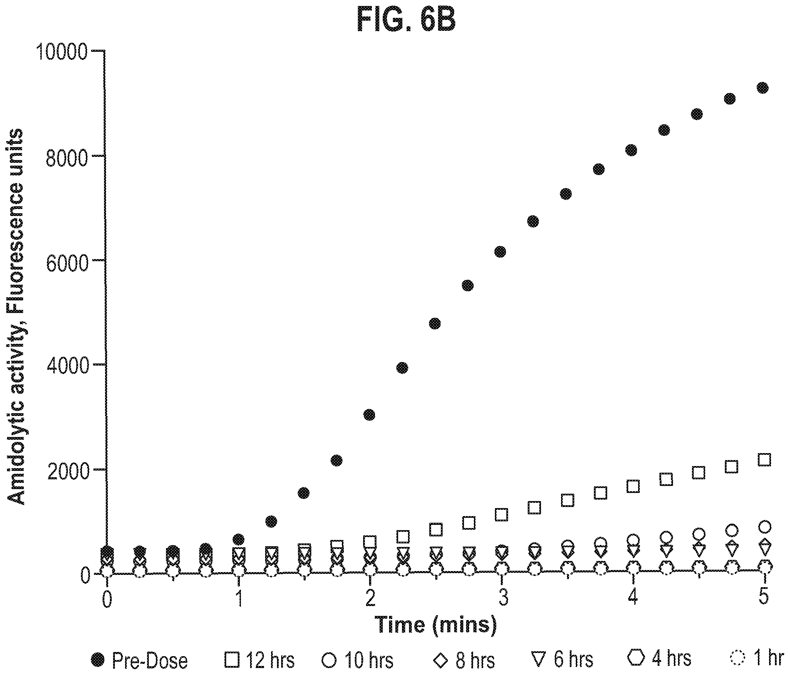

The kinetic fluorescent measurements from the undiluted plasma enzyme assay can be plotted as assay progression curves (Figures 6A and 6B). These curves highlight that the compound of Formula A not only has an inhibitory effect on enzyme activity but also increases the time until appearance of catalytic activity during contact system activation (lag time). At early time points post-dose administration, plasma samples did not display detectable catalytic activity even after prolonged activation with the potent activator DXS. In this test, the subject was administered with 600 mg dose in a tablet formulation.

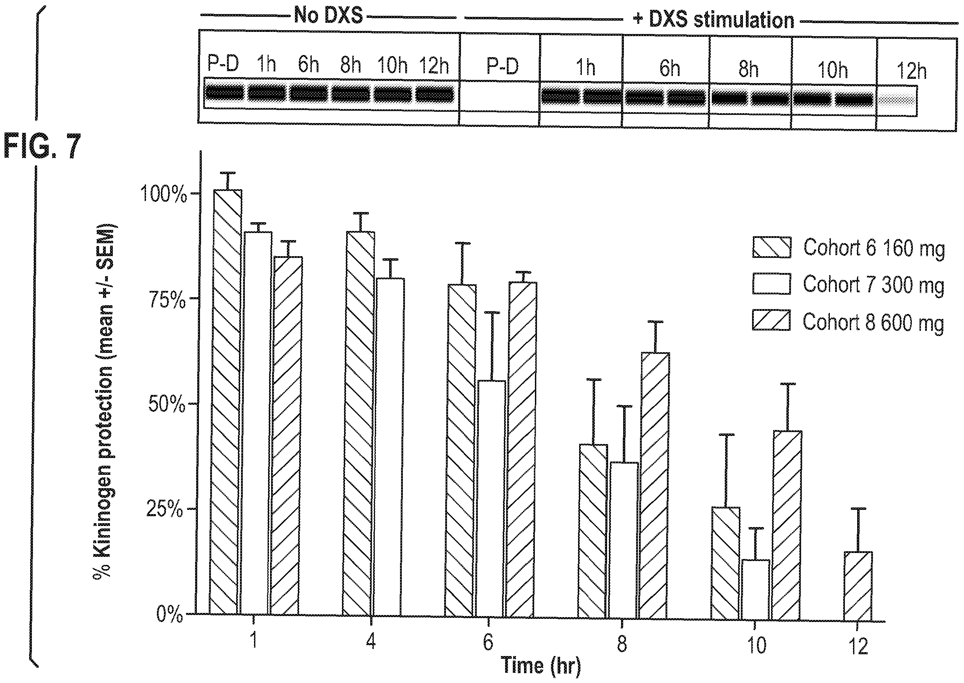

Figure 7 shows the mean percent HK protection in DXS-activated undiluted plasma (SAD cohort 6 (160 mg), 7 (300 mg) and 8 (600 mg)). As shown, all three doses of the compound of Formula A were able to inhibit plasma kallikrein catalytic activity above 90% for a period of time. The duration of these PD effects was dose proportional. The compound of Formula A is shown to protect HK from DXS-activated cleavage in undiluted plasma for at least 10 hr following a single 600 mg dose.

In Figure 7, the representative WES system gel image was generated in duplicate undiluted plasma samples +/- DXS activation from a single subject in cohort 8 who received 600 mg of the compound of Formula A compared with pre-dose (P-D).

In Figure 7, HK cleavage was evaluated following DXS activation of undiluted plasma samples at selected time points from cohorts 6 to 8. Data are expressed as Mean +/- SEM, n=6.

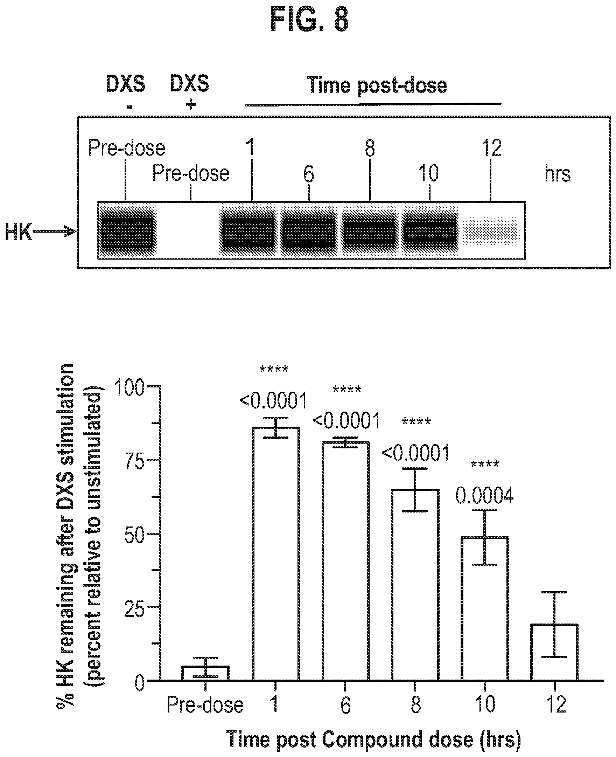

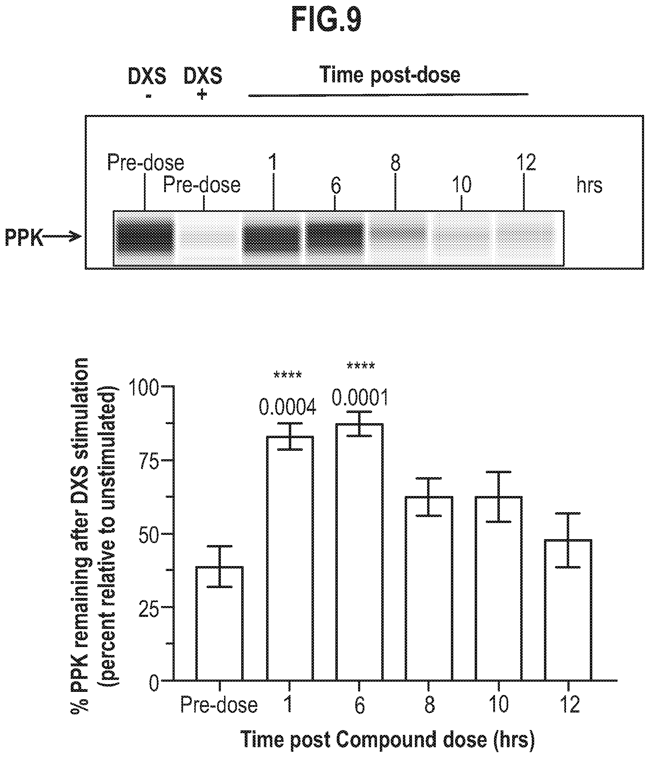

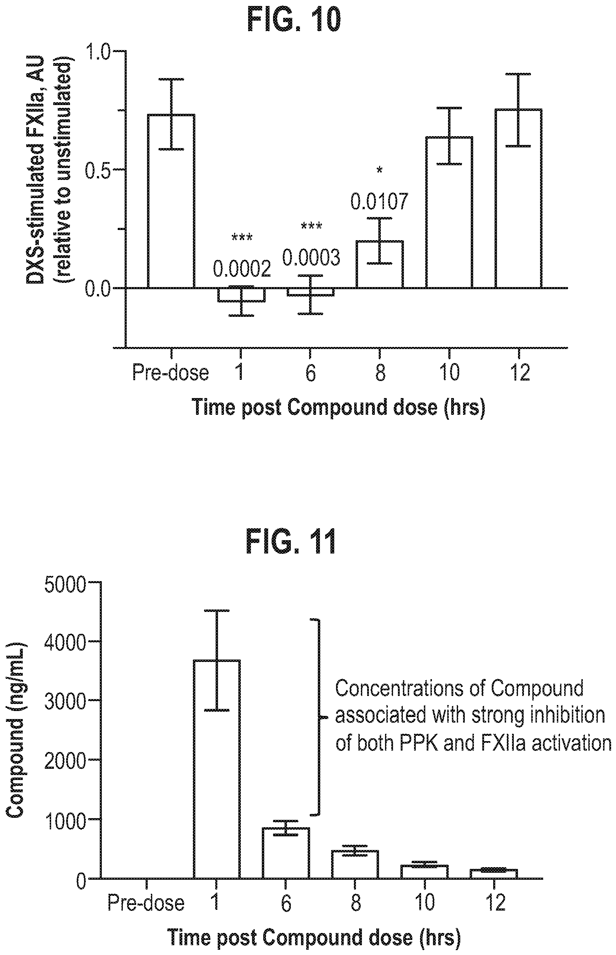

To assess whether the compound of Formula A also reduced the generation of plasma kallikrein and Factor XIIa, immunoassays were used to quantify levels of contact system proteins in DXS-activated plasma at pre-dose and up to 12 hrs post-dose of 600 mg orally administered in capsules. The results from these assays are shown in Figures 8 to 11 and shows that the compound of Formula A not only reduces HK cleavage but also reduces PPK cleavage and reduces the generation of FXlla. These results suggest that the compound of Formula A inhibits the contact activation system *via* interruption of the positive feedback loop mediated by PKa stimulated activation of FXII.

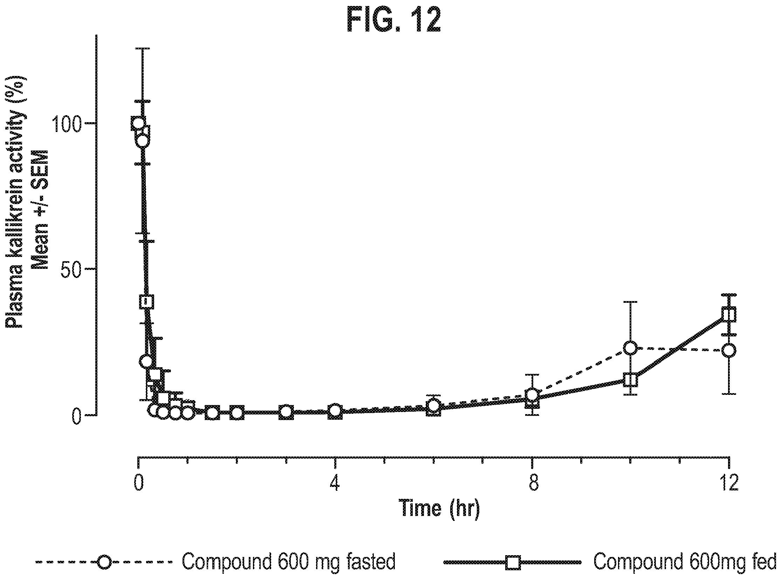

Figure 12 shows that no significant food-effect was observed on the pharmacodynamic (PD) profile of a 600 mg tablet provided in fed and fasted state. As can be seen, the PD effects are rapidly observed in fed and fasted state with plasma kallikrein inhibition of >90% achieved by 30 minutes in both states.

No serious adverse events were reported in the phase I trial. There were also no tolerability signals. No subjects withdrew from the trial.

These data demonstrate that the compound of Formula A has an inhibitory effect on the bradykinin and contact activation systems. As discussed above, these pharmacodynamic effects are implicated in disorders such as HAE. These data also show that the compound of Formula A has a pharmacokinetic profile suitable for oral administration.

### Example 5 - Immunoassays investigating the compound of formula A in the protection of high molecular weight kininogen (HK) from PKa-mediated cleavage in HAE and control plasma

### Method:

High molecular weight kininogen (HK) cleavage in undiluted citrated human plasma was induced by contact system activation with dextran sulfate (DXS, Sigma #31395-10G; 6.25 µg/ml) on wet ice. Pooled normal (CONTROL) human plasma (VisuCon-F Frozen Normal Control plasma) was purchased from Affinity Biologicals Inc. A working stock of 10mM of the compound of formula A ("the compound") in DMSO was prepared and diluted in 1X PBS to the respective final concentrations described. HAE plasma was obtained from HAE subjects (n=6) and C1-inhibitor deficiency was confirmed by western blotting. Protection of HK from PKa mediated cleavage in DXS-stimulated whole undiluted plasma was then determined by two methods; traditional western blotting and a semi-automated capillary-based immunoassay.

*Western blotting:* SDS-PAGE gel electrophoresis was done using 7.5% Criterion TGX Precast gels (Bio-rad). Transfer was made onto Immobilon-FL PVDF membrane. Image analysis was performed using the LICOR imaging system. Mouse monoclonal anti-human HK antibody (MAB15692, R&D systems) was used for traditional immunoblotting.

### Capillary-based immunoassay on the WES System (ProteinSimple):

**Preparation of samples:** Combine one part 5× fluorescent master mix with four parts of the 1:200 plasma sample. Vortex to mix. Heat the samples + fluorescent master mix and the biotinylated ladder at 95°C for 5 minutes, vortex, and load onto the WES plate. Monoclonal anti-human HK antibody was used for this chemiluminescence-based detection method using the Wes System (ProteinSimple).

**Analysis:** Collect the peak area measurement obtained in the Compass software (cbz file) for the full-length HK molecular weight of the respective time-point sample with DXS-induced activation. The peak area is defined as the area calculated for the spectral peak profile for HK. To measure the plasma kallikrein inhibition by the compound, the percent full-length HK detected was calculated.

### Results:

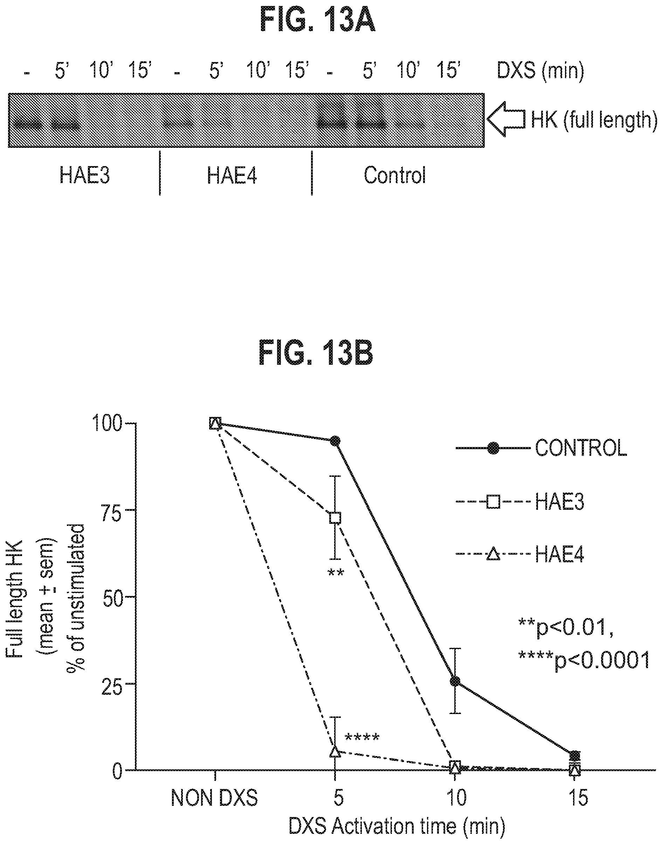

Figures 13A and 13B show the time course of dextran sulfate-activated cleavage of HK in HAE whole undiluted plasma determined using western blotting, and a representative blot.

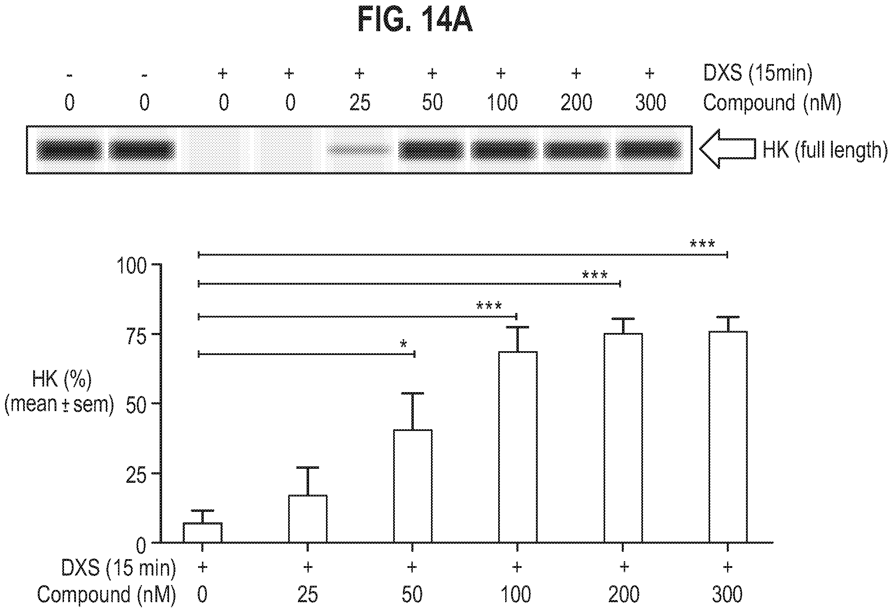

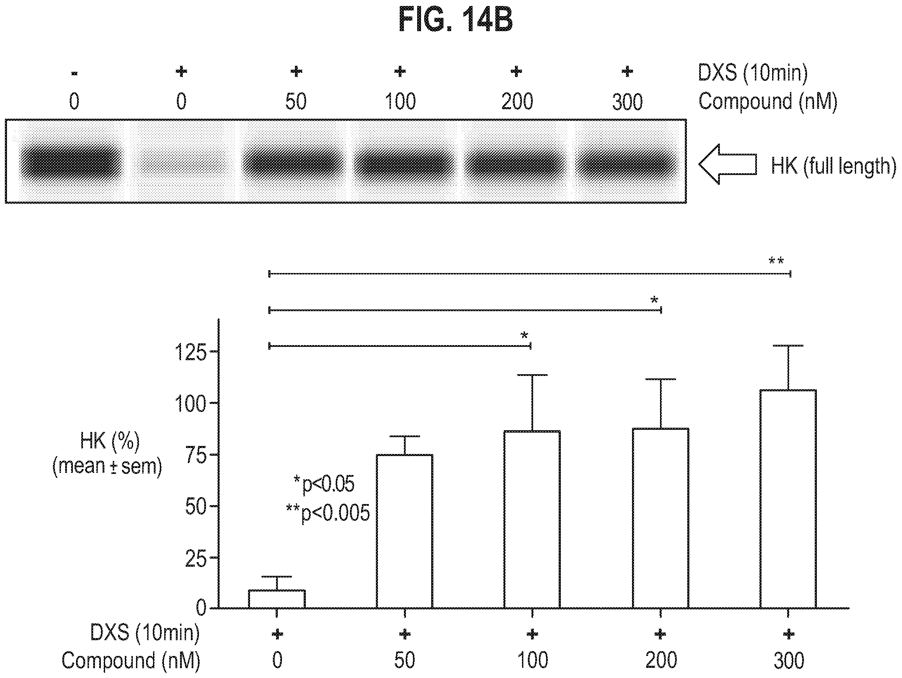

Figures 14A and 14B shows a representative WES system gel image and that the compound of Formula A provides dose dependent protection against HK cleavage in both HAE and healthy control plasma stimulated with dextran sulfate determined by capillary-based immunoassay using the WES system.

### Example 6 - Phase 2 study of the compound of Formula A

Aim: To evaluate the efficacy and safety of the compound of Formula A in the on-demand treatment of angioedema attacks in adult subjects with hereditary angioedema type I or II.

### Methods:

The study is a randomized, double-blind, placebo-controlled, phase 2, cross-over clinical trial evaluating the efficacy and safety of the compound of formula A ("the compound"), an oral plasma kallikrein inhibitor, in the on-demand treatment of angioedema attacks in adult subjects with hereditary angioedema type I or II (EudraCT number: 2018-004489-32).

### Objectives:

### Primary Objective:

- To investigate the efficacy of the compound compared to placebo in halting the progression of a peripheral or abdominal attack of hereditary angioedema (HAE).

### Secondary Objectives:

- To investigate the safety and tolerability of the compound.
- To investigate the pharmacokinetic (PK) profile of the compound when taken during the intercritical period between HAE attacks.
- To investigate the pharmacodynamic (PD) profile of the compound in reducing the concentration of residual cleaved high molecular weight kininogen (HK) during the intercritical period between HAE attacks.
- To investigate the PD profile of the compound in reducing activated plasma enzyme activity during the intercritical period between HAE attacks.

### Setup:

This is a phase 2, two-part, two-sequence, two-period (2x2) cross-over clinical trial. Subjects with HAE type I or II will be recruited through HAE treatment centres in Europe and US.

In Part 1, subjects will receive a single oral dose of 600 mg of the compound to investigate the safety, PK and PD of the compound during the intercritical period between HAE attacks.

Eligible adult subjects ≥18 years old will undergo a screening assessment for study inclusion, receive study drug, followed by a 4h, in-clinic, safety and PK / PD assessment.

In Part 2, the subjects will be randomized 1:1 to 2 treatment sequences. This part of the study will be conducted away from the clinic or hospital. In Sequence 1 (study arm 1) subjects will receive a single dose of 600 mg of the compound to treat the first eligible HAE attack. Following resolution of this attack, subjects will receive a second single dose of placebo to treat the second eligible HAE attack.

In Sequence 2 (study arm 2) subjects will receive a single dose of placebo to treat the first eligible HAE attack. Following resolution of this attack, subjects will receive a second single dose of 600 mg of the compound to treat the second eligible HAE attack.

A minimum of 48-hour washout period is required between each dose of study drug.

Laryngeal or facial attacks are not eligible for treatment. HAE attacks must be treated within the first hour of onset and before reaching severe on the global attack severity scale. Subjects must also be able to identify the start of a HAE attack. Upon onset of the eligible HAE attack, subjects will notify the dedicated study physician or qualified designee with a description of the HAE attack. The dedicated study physician or qualified designee will confirm eligibility of the HAE attack and agree to study drug being administered. HAE attacks require documentation, on the Subject Diary, of attack location, attack symptoms, time of onset, attack severity, and time of last substantial meal prior to dosing. Subjects will take study drug, as instructed, and will complete timed assessments of their HAE attack symptoms for a 48h period as documented below in Table 3. The dedicated study physician or qualified designee will contact the subject within 24h of the eligible HAE attack to confirm the subject's safety and wellbeing. Subjects will be instructed to contact the dedicated study physician or qualified designee in case of any safety concerns. In the case of hypersensitivity, subjects are to contact the dedicated study physician or qualified designee or contact the nearest emergency service. The dedicated study physician or qualified designee will be available 24h/day and 7 days/week to receive subject calls.

**Table 3: Frequency of Subject Assessment**

| **Time Period following Study Drug Administration** | **Frequency of Subject Assessment*** | **Allowed Time Window for Assessment** |
|---|---|---|
| 0h - 4h | Every 30 min | None |
| 4h - 12h | Every 1h | +/- 15 min |
| 12h - 24h | Every 3h | +/- 30 min |
| 36h | Once | +/- 60 min |
| 48h | Once | +/- 60 min |

| | | |
|---|---|---|
| **in the event that **conventional attack treatment is used**, the subject should perform assessments every 30 min for 4 h following first administration of conventional attack treatment. After this, the subject should revert back to original frequency of assessments based on time of study drug administration.* | | |

Subjects will return to the clinic following the first HAE attack, prior to the second HAE attack, to undergo safety checks including adverse event (AE) reporting, vital sign recording, and Subject Diary review.

Once two HAE attacks have been treated in Part 2, the subject will return to the clinic to undergo final safety checks including AE reporting, vital sign recording and blood sampling for laboratory safety measurements.

Conventional attack treatment is permitted after 4h, or earlier as warranted, following study drug intake, provided HAE attack symptoms are judged severe enough by the subject to require treatment as per the subject's usual treatment regimen, or are deemed ineligible for study drug treatment, or are associated with laryngeal or facial symptoms. Prior to use of conventional attack treatment, subjects will notify the dedicated study physician or qualified designee who will confirm conventional treatment is appropriate per protocol and subject report of symptom severity. Subjects are permitted to treat their HAE attacks with their conventional attack treatment (pdC1INH or rhC1INH intravenous [iv] or icatibant).

### Investigational Medicinal Product:

The compound of formula A - 100 mg film-coated tablet. These contain the following excipients:
microcrystalline cellulose, croscarmellose sodium, povidone, magnesium stearate; the aesthetic coating contains hypromellose, lactose monohydrate, titanium dioxide and triacetin.

Placebo to the compound 100 mg film-coated tablet. These contain microcrystalline cellulose, colloidal silicon dioxide, sodium starch glycolate, and sodium stearyl fumarate and are film-coated; the aesthetic coating contains hypromellose, lactose monohydrate, titanium dioxide and triacetin.

No study drug dose modifications are allowed in this study.

### Number of Subjects:

Approximately 60 subjects will be enrolled into the study to ensure 50 subjects complete the study.

### Population:

The study population will include male and female subjects 18 years of age or older with HAE type I or II.

### Inclusion Criteria:

1. Male or female adult subjects 18 years of age and older.
2. Confirmed diagnosis of HAE type I or II at anytime in the medical history:
   a. Documented clinical history consistent with HAE (subcutaneous or mucosal, nonpruritic swelling episodes without accompanying urticaria) AND
   b. C1-esterase inhibitor (C1-INH) antigen or functional level <40% of the normal level. Subjects with antigen or functional C1-INH level 40-50% of the normal level may be enrolled if they also have a C4 level below the normal range and a family history consistent with HAE type I or II.
3. At least 3 documented HAE attacks in the past 93 days, as supported by medical history.
4. Access to and ability to use conventional attack treatment for attacks of HAE.
5. Adequate organ functions as defined below:
   a. Hemoglobin within normal range;
   b. International normalized ratio (INR)< 1.2;
   c. Activated partial thromboplastin time (aPTT) ≤ upper limit of normal (ULN);
   d. Creatinine < 1x ULN;
   e. Creatinine clearance (CrCl) ≥ 60 mL/min;
   f. Alanine aminotransferase (ALT) ≤ 2x ULN;
   g. Aspartate aminotransferase (AST) ≤ 2x ULN;
   h. Total bilirubin ≤ 1.5x ULN;
   i. Leucocytes ≤ 1.5x ULN;
   j. Thrombocytes ≤ 1.5x ULN.
6. Female of childbearing potential must agree to use highly effective birth control from the Screening visit until the end of the trial follow-up procedures.

Highly effective methods of birth control include:
a. Progestogen-only hormonal contraception associated with inhibition of ovulation: oral / injectable / implantable.

(Hormonal contraception that contains estrogen is excluded per exclusion criterion 3).
b. Intrauterine device (IUD).
c. Intrauterine hormone-releasing system (IUS).
d. Bilateral tubal occlusion.
e. Vasectomised partner (provided that the partner is the sole sexual partner of the female subject of childbearing potential and that the vasectomised partner has received medical assessment of the surgical success).
f. Sexual abstinence (this method is not acceptable in Switzerland).
**Note:** Sexual abstinence will only be considered a highly effective method if it is defined as refraining from heterosexual intercourse. The reliability of sexual abstinence needs to be evaluated in relation to the duration of the clinical trial and the preferred and usual lifestyle of the subject.
7. Females of non-childbearing potential, defined as surgically sterile (status post hysterectomy, bilateral oophorectomy, or bilateral tubal ligation) or post-menopausal for at least 12 months, do not require contraception during the study.
8. Males with female partners of childbearing potential must agree to be abstinent or else use a highly effective method of birth control as defined in inclusion criterion 6 from the Screening visit until the end of the trial follow-up procedures.
9. Provide signed informed consent and are willing and capable of complying with study requirements and procedures.

### Exclusion criteria:

1. Any concomitant diagnosis of another form of chronic angioedema, such as acquired C1 inhibitor deficiency, HAE with normal C1-INH (also known as HAE type III), idiopathic angioedema, or angioedema associated with urticaria.
2. Current use of C1INH, androgens, lanadelumab or tranexamic acid for HAE prophylaxis.
3. Use of angiotensin-converting enzyme (ACE) inhibitors or any estrogen-containing medications with systemic absorption (such as oral contraceptives or hormonal replacement therapy) within 93 days prior to initial study treatment.
4. Use of androgens (e.g. stanozolol, danazol, oxandrolone, methyltestosterones, testosterone) or antifibrinolytics within 30 days prior to initial study treatment.
5. Use of lanadelumab within 10 weeks prior to initial study treatment.
6. Use of strong CYP3A4/CYP2C9 inhibitors and inducers during participation in the trial.
   **Note:** These medications include but are not limited to the following: cobicistat, conivaptan, itraconazole, ketoconazole, posaconazole, voriconazole, ritonavir, boceprevir, telaprevir, troleandomycin, clarithromycin, carbamazepine, enzalutamide, mitotane, phenytoin, phenobarbital, fluconazole, isoniazid, metronidazole, paroxetine, sulfamethoxazole, rifampicin, St. John's Wort, diltiazem, idelalisib, nefazodone and nelfinavir.
7. Clinically significant abnormal electrocardiogram (ECG) at Visit 1 and pre-dose at Visit 2. This includes, but is not limited to, a QTcF > 470 msec (for women) or > 450 msec (for men), a PR > 220 msec or ventricular and/or atrial premature contractions that are more frequent than occasional and/or occur as couplets or higher in grouping.
8. Any clinically significant history of angina, myocardial infarction, syncope, clinically significant cardiac arrhythmias, left ventricular hypertrophy, cardiomyopathy, or any other cardiovascular abnormality.
9. Any other systemic dysfunction (e.g., gastrointestinal, renal, respiratory, cardiovascular) or significant disease or disorder which, in the opinion of the Investigator, would jeopardize the safety of the subject by taking part in the trial.
10. History of substance abuse or dependence that would interefere with the completion of the study, as determined by the Investigator.
11. Known lactose allergy or intolerance.
12. Known hypersensitivity to the compound or placebo or to any of the excipients.
13. Participation in an interventional investigational clinical study within 93 days or within 5 half-lives of the last dosing of investigational drug (whichever is longer) prior to initial study treatment.
14. Any pregnant or breast-feeding subject.

### Assessments:

*Part 1:* Blood samples for PK and PD measurements will be collected at the following timepoints:
Pre-dose (0h), 15 min, 30 min, 45 min, 1h, 1.5h, 2h, 3h, and 4h post-dose. Vital signs (systolic blood pressure [SBP], diastolic blood pressure [DBP], pulse rate [PR], respiratory rate [RR] and body temperature) will be measured at pre-dose (0h), 1h, and 4h post-dose. Samples for post-treatment safety laboratory assessments will be taken with the 4h PK / PD samples.

*Part 2:* Following study drug intake, subject assessments of overall HAE attack severity and change in HAE attack severity will take place for a 48h period as documented in Table 3 above.

### Efficacy Variables:

Time to use of conventional attack treatment will be assessed. The subject diary will capture the efficacy endpoints including time to use of conventional attack treatment and HAE attack severity.

Overall HAE attack severity will be assessed on a 5-point Likert scale (5LS) scored as none, mild, moderate, severe and very severe.

Change in HAE attack severity will be assessed using a 7-point transition question (7TQ), scored as Much better / Better / A little better / No change / A little worse / Worse / Much worse.

The type of HAE attack symptoms (abdominal pain, skin pain and skin swelling) will each be assessed on a 100 mm visual analogue scale (VAS) anchored at 0 (none) and 100 (very severe).

### Safety Variables:

- AEs, including serious adverse events (SAEs).
- Laboratory test results (clinical chemistry, hematology, coagulation, and urinalysis).
- Vital signs (SBP, DBP, PR, RR, body temperature).
- Physical examination findings.
- ECG results.
- Pregnancy test (female subjects of child-bearing potential).

### Criteria for Evaluation of Efficacy

### Primary Efficacy Endpoints:

- Time to use of conventional attack treatment.

### Secondary Efficacy Endpoints:

- Proportion of HAE attacks that progress by one level or more on the 5LS or that require conventional attack treatment within 12h of study drug.
- Time between treatment and (1) progression of global attack severity on the 5LS by one level or more, or (2) use of conventional attack treatment, whichever comes first within 12h.

### Exploratory Endpoints:

- Cumulative global attack severity on the 5LS following study drug expressed as area under the curve (AUC) for the compound 600 mg vs. placebo.
- Proportion of HAE attacks that require conventional attack treatment.
- Proportion of HAE attacks that are rated "worse" or "much worse" on the TQ.
- Proportion of HAE attacks that are rated "better" or "much better" on the TQ.
- Time from study drug administration to complete HAE attack resolution (rating of none) on global attack severity scale (5LS).
- Time to HAE attack being rated worse or much worse on the TQ.
- Time to HAE attack being rated better or much better on the TQ.

### General Statistical Methods and Types of Analyses

### Analysis Sets:

- Safety set (SAF): Subjects who have taken at least one dose of study drug (including the study drug dose in Part 1).
- Full analysis set (for efficacy) (FAS): All randomized subjects who received both doses of study drug in Part 2.
- Per protocol set (for efficacy) (PPS): Randomized subjects in Part 2 who received the compound both doses of study drug in Part 2 and have no major protocol deviations.
- PK / PD analysis set: All subjects for whom PK / PD samples were taken in Part 1.

### Sample size:

A sample size of 50 subjects (25 per sequence) is proposed to provide 90% power for testing at the 5% alpha level (2-sided) for the primary endpoint of time to use of conventional attack treatment. This sample size has been derived based upon an assumption that 40% of subjects will use conventional attack treatment while on the control arm while 10% will use conventional attack treatment on the experimental arm and that within subject data has minimal correlation. The assumption of minimal correlation should be a conservative assumption with respect to sample size. Approximately 60 subjects will be enrolled to ensure that 50 subjects complete the study.

An oversampling by 20% (10 subjects) is proposed to account for subjects that may not complete both treatment periods due to infrequent or ineligible HAE attacks or for subjects who discontinue the trial early, for whatever reason. Thus, study enrolment will be considered sufficient to address the primary efficacy hypothesis after 50 subjects have completed both treatment periods. Since further exposure is not required and could be considered unnecessary, ongoing subjects who have not completed both periods will be asked to return to the study site and complete Visit 4 (Early Discontinuation visit). Data from all subjects, complete and incomplete, will be analyzed in the safety set.

### General Considerations:

Individual subject data will be presented in subject data listings. Appropriate descriptive statistics will be calculated for continuous and categorical data and summarized in tabular format.

### Sample Analyses:

AEs will be coded using the Medical Dictionary for Regulatory Activities (MedDRA) dictionary (v21.0 or higher) and classified by preferred term and system organ class (SOC). Listings of treatment-emergent adverse events (TEAEs), serious TEAEs, and TEAEs causing premature discontinuation will be provided by sequence group, and further classified by TEAE severity and relationship to study drug.

### Efficacy Analyses:

### Primary Endpoint

The primary endpoint, time to use of conventional attack treatment, will be analyzed using a generalization of Gehan's test proposed by Feingold and Gillespie (1996) (Crossover trials with censored data. Statistics in Medicine 1996; 15(10): 953-967) to reflect the repeat measures on each subject. Subjects will be treated as censored if no worsening occurs within 12h of study drug.

### Secondary Endpoints

The proportion of HAE attacks that worsen by one level or more on the 5LS or that require conventional attack treatment within 12h of study drug will be analyzed using Prescott's test (1981) (The comparison of success rates in cross-over trials in the presence of an order effect. Applied Statistics 1981; 30: 9-15) to compare the treatment arms.

A similar approach to that used for the primary endpoint will be followed for the analysis of the time between study drug and HAE attack worsening by one level or more on the 5LS or use of conventional attack treatment, whichever comes first within 12h. In addition to the tests described above, descriptive statistics will be presented for the primary, secondary and exploratory endpoints, in each case comparing the compound to placebo, such as:
- Cumulative global attack severity on the 5LS following study drug expressed as AUC for the compound 600 mg vs. placebo.
- Proportion of HAE attacks that require conventional attack treatment.
- Proportion of HAE attacks that are rated "worse" or "much worse" on the TQ.
- Proportion of HAE attacks that are rated "better" or "much better" on the TQ.
- Time from study drug administration to complete HAE attack resolution (rating of none) on global attack severity scale (5LS).
- Time to HAE attack being rated worse or much worse on the TQ.
- Time to HAE attack being rated better or much better on the TQ.

### PK Analysis:

Non-compartmental PK parameters will include maximum concentration in plasma (Cmax), time to reach Cmax in plasma (tmax), and area under the curve from time 0 to last sample (AUC0-t). Compartmental PK modelling will describe the PK of the compound and generate underlying Cmax, tmax, AUC, apparent clearance (CL/F), apparent volume of distribution (Vd/F) and estimated terminal elimination half-life (t½).

The PK parameters of the compound will be determined from the individual concentration versus time data using Phoenix WinNonlin. In case of a deviation from the theoretical time, the actual time of blood sample will be used in the calculation of the derived PK parameters. Individual concentrations and derived PK parameters of the compound in plasma will be listed and summarized for each treatment. Individual and geometric mean concentration-time data will be plotted on linear and semi-logarithmic scales.

### PD Analysis:

The compound's effect on plasma kallikrein (PKa) activity will be analyzed using two exploratory measures of PKa enzyme activity in plasma:
- An assay to determine inhibition of exogenously activated plasma kallikrein enzyme activity from plasma samples obtained before and after receiving the compound.
- An assay to measure the level of protection of cleavage of high molecular weight kininogen (HK) substrate (contained in whole plasma) from plasma kallikrein enzyme activity.

The PD will be summarized for each treatment. Individual and mean data will be provided as a report addendum located in the appendix of the final Clinical Study Report.

### Preliminary PK data from Part 1 of the study:

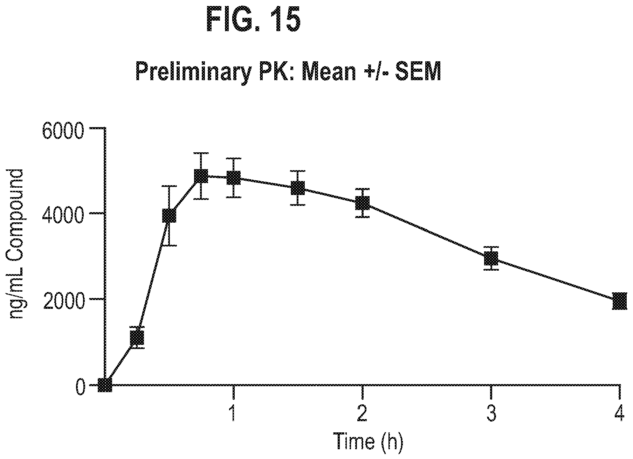

At the time of filing this application, preliminary PK data from 27 HAE patients have been collated and analysed, and are shown in Table 4 and Figure 15.

**Table 4**

| **Dose** | **Cmax (ng/mL)** |
|---|---|
| | **Mean (95% CI)** |
| | **n=27** |
| 600 mg | 5907 (4913, 6901) |

Thus, these preliminary results show that the compound of Formula A demonstrates a pharmacokinetic profile that is suitable for on-demand oral administration in HAE patients. The study is ongoing at the time of filing.

### Example 7 - Phase 1 multiple dose study in healthy adult subjects

Aim: To evaluate the safety, tolerability, pharmacokinetics, and the change from baseline in QTc following administration of the compound formulated as 100 mg film coated tablets in healthy adult subjects.

### Primary Objective:

- To investigate the safety and tolerability of multiple doses of the compound.

### Secondary Objectives:

- To investigate the pharmacokinetics (PK) of multiple doses of the compound.
- To evaluate the effects of the compound on ECG parameters, including concentration-QTc relationship, following administration of the compound 100 mg Film Coated Tablets (KalVista Pharmaceuticals) to healthy adult subjects.

### Exploratory Objectives:

- To investigate the pharmacodynamics (PD) of multiple doses of the compound.

### Methods:

This is a phase 1, double-blind, placebo-controlled, multiple-dose, multiple-cohort study to evaluate safety and tolerability of the compound as well as of the ECG effects of the compound formulated as 100 mg Film Coated Tablets in healthy adult male and female subjects.

Four (4) cohorts are planned for evaluation. Cohorts 1, 2 and 3 will include 8 subjects each. Cohort 4 will include 18 subjects. Every attempt will be made to include an equal number of male and female subjects in each cohort.

During the study, oral doses of 600 mg of the compound as Film Coated Tablets (six 100 mg tablets) or 6 matching placebo tablets will be administered once every 8 hours (Cohort 1) every 4 hours (Cohort 2), or every 2 hours (Cohort 3 and 4) to healthy adult male and female subjects up to a total dose of 1800 mg. In Cohorts 1, 2 and 3, 6 subjects will receive the compound as 100 mg Film Coated Tablets and 2 subjects will receive the placebo for a total of 8 subjects per cohort. In Cohort 4, 12 subjects will receive the compound as 100 mg Film Coated Tablets and 6 subjects will receive the placebo for a total of 18 subjects.

Progression from Cohort 1 to Cohort 2 and Cohort 2 to Cohort 3 will occur after review of the safety data (labs, vital signs, safety ECGs, and adverse events) captured during the conduct of Cohort 1 and Cohort 2. Progression to Cohort 4 will occur after review of the safety data and pharmacokinetic data from Cohort 3. The pharmacokinetic data from Cohort 3 will be reviewed to ensure that the Cmax of the 3rd dose is high enough to support the evaluation of the change in the QTc interval from baseline.

A Holter monitor will be attached to each subject in order to continuously record ECGs. The monitor will be attached 1 hour before the first dose and will remain attached until after the final blood sample collection. The electrodes for the Holter monitor will be checked by a member of the clinic staff at appropriate intervals to ensure they are attached.

Blood samples will be collected at pre-dose, at intervals after the first dose, and at intervals over 24 hours after the final (third) dose (40 hours from the initial dose in Cohort 1, 32 hours from the initial dose in Cohort 2, 28 hours from the initial dose in Cohorts 3 and 4) in each cohort. Subjects will be confined to the clinical facility from at least 10 hours before dosing until after the final blood sample collection in each study cohort and will return to the clinic 5 to 7 days after the final dose for safety evaluations.

The pharmacokinetics of the compound will be measured by a fully validated analytical procedure and the pharmacodynamic effect on plasma kallikrein inhibition enzyme activity will be evaluated by an exploratory pharmacodynamic assessment.

Statistical analysis will be performed to evaluate the relationship between plasma drug concentrations and the change from baseline in ECG effects of the test formulation.

### Treatment administration

### Cohort 1

The subjects will receive the test or placebo treatment every 8 hours over a 16-hour period (3 administrations of: 6 × 100 mg of the compound as 100 mg Film Coated Tablets or placebo dose administrations at 0, 8, and 16 hours, total dose of 1800 mg of the compound or placebo) according to a two-treatment randomization schedule under direct observation. Each dose will be administered with 240 mL of room temperature water. Subjects will be instructed to swallow the tablets whole without chewing or biting. Any subject who bites or chews the tablets will be dropped from the study. Immediately after dosing a mouth check will be performed

### Cohort 2

The subjects will receive the test or placebo treatment every 4 hours over an 8-hour period (3 administrations of: 6 × 100 mg of the compound as 100 mg Film Coated Tablets or placebo dose administrations at 0, 4, and 8 hours, total dose of 1800 mg of the compound or placebo) according to a two-treatment randomization schedule under direct observation. Each dose will be administered with 240 mL of room temperature water. Subjects will be instructed to swallow the tablets whole without chewing or biting. Any subject who bites or chews the tablets will be dropped from the study. Immediately after dosing a mouth check will be performed to ensure that the tablets were swallowed whole without chewing or biting.

### Cohort 3 and 4

The subjects will receive the test or placebo treatment every 2 hours over a 4- hour period (3 administrations of: 6 × 100 mg of the compound as 100 mg Film Coated Tablets or placebo dose administrations at 0, 2, and 4 hours, total dose of 1800 mg of the compound or placebo) according to a two-treatment randomization schedule under direct observation. Each dose will be administered with 240 mL of room temperature water. Subjects will be instructed to swallow the tablets whole without chewing or biting. Any subject who bites or chews the tablets will be dropped from the study. Immediately after dosing a mouth check will be performed to ensure that the tablets were swallowed whole without chewing or biting.

All subjects will fast (except water) for at least 8 hours before the first dosing. After initial dosing, subjects will continue to fast until at least 6 hours after the first dose.

### Method of Assigning Subjects to Treatment Groups:

### Cohort 1, 2 and 3

Subjects will be randomized such that 6 subjects will receive the test product and 2 subjects will receive the placebo. As a safety measure, a sentinel dosing scheme will be incorporated for each cohort, in which one subject will receive the test product and one subject will receive the placebo product followed by the remainder of the cohort.

### Cohort 4

Subjects will be randomized such that 12 subjects receive the test product and 6 subjects receive the placebo.

The randomization schedule will be generated prior to the first dosing cohort using SAS^{®}, Version 9.4 or higher.

### Results:

No serious adverse events were reported during the study and no subjects were discontinued because of an AE. All reported adverse events were considered "mild" in severity and had an outcome of "recovered/resolved" at the end of the study.

No clinically relevant effects on the studied ECG parameters were identified.

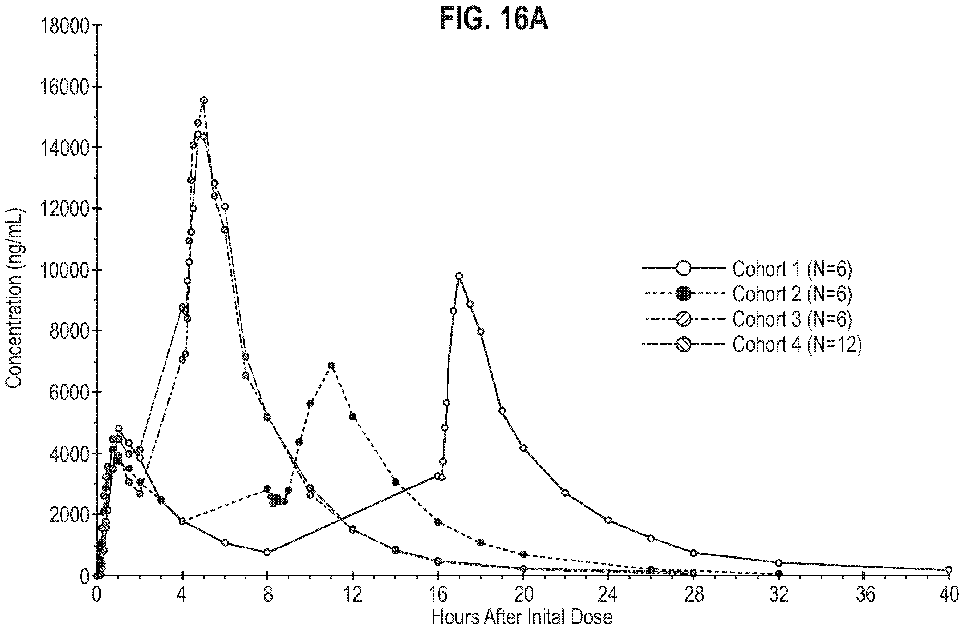

Figure 16A shows the mean plasma concentrations of the compound of Formula A after the initial dose for each cohort.

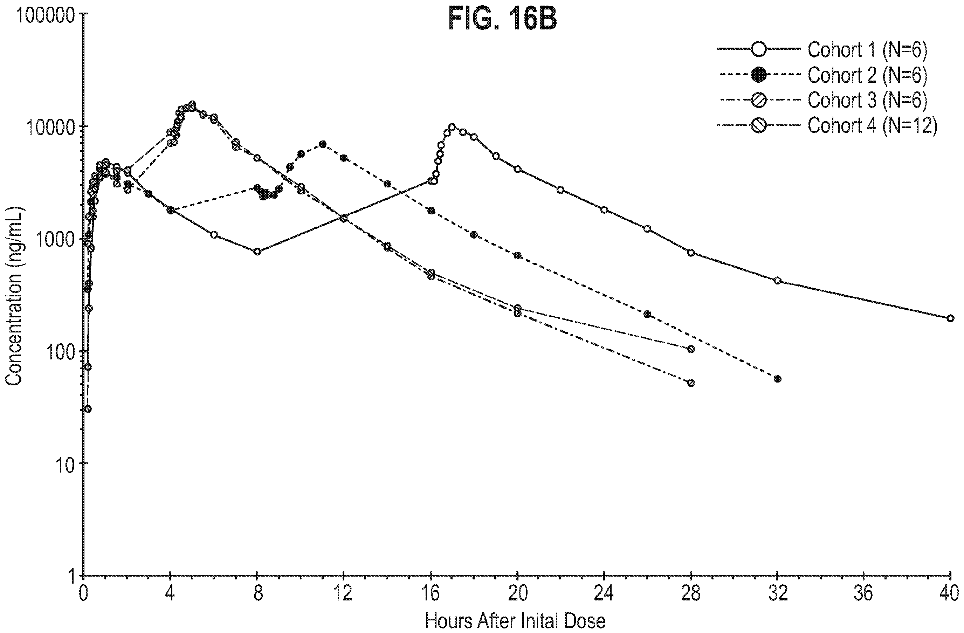

Figure 16B shows the mean plasma concentrations (semi-logarithmic scale) of the compound for formula A for each cohort.

These data demonstrate that the compound of Formula A has a pharmacokinetic profile suitable for oral administration when administered in multiple dosage amounts. The results further suggest that the compound of Formula A can be dosed safely at regular intervals.

## Claims

1. A compound of Formula A (or a pharmaceutically acceptable salt and/or solvate thereof) for use in treating hereditary angioedema (HAE) on-demand comprising: orally administering the compound of Formula A (or a pharmaceutically acceptable salt and/or solvate thereof) to a patient in need thereof on-demand,

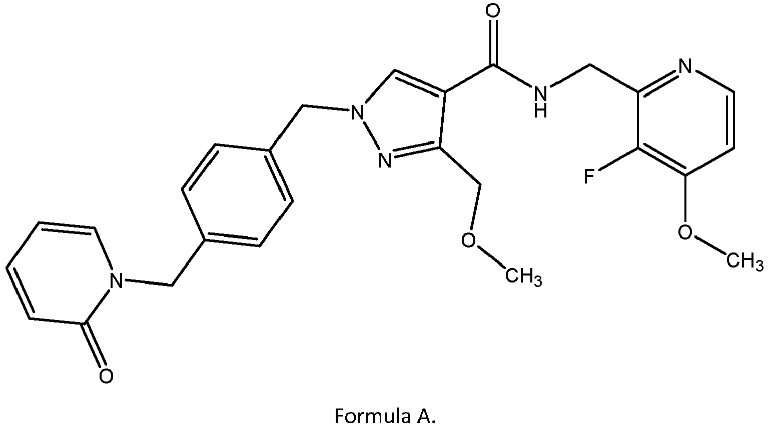

2. The compound of Formula A (or a pharmaceutically acceptable salt and/or solvate thereof) for use according to claim 1,
wherein the compound of Formula A (or a pharmaceutically acceptable salt and/or solvate thereof) is for use in treating an acute attack of hereditary angioedema (HAE) on-demand and is orally administered on-demand upon recognition of a symptom of an acute HAE attack.

3. The compound of Formula A (or a pharmaceutically acceptable salt and/or solvate thereof) for use according to claim 2,
wherein the symptom of an acute HAE attack recognised is at least one of: swelling of tissues; fatigue; headache; muscle aches; skin tingling; abdominal pain; nausea; vomiting; diarrhoea; difficulty swallowing; hoarseness; shortness of breath; and/or mood changes.

4. The compound of Formula A (or a pharmaceutically acceptable salt and/or solvate thereof) for use according to any of claims 2 or 3,
wherein the compound of Formula A (or a pharmaceutically acceptable salt and/or solvate thereof) is orally administered on-demand within 1 hour of the symptom of an acute HAE attack being recognised.

5. The compound of Formula A (or a pharmaceutically acceptable salt and/or solvate thereof) for use according to any of claims 2 to 4,
wherein the compound of Formula A (or a pharmaceutically acceptable salt and/or solvate thereof) is orally administered on-demand within 30 minutes, within 20 minutes, within 10 minutes, or within 5 minutes of the symptom of an acute HAE attack being recognised.

6. The compound of Formula A (or a pharmaceutically acceptable salt and/or solvate thereof) for use according to any of claims 2 to 5,
wherein the compound of Formula A (or a pharmaceutically acceptable salt or solvate thereof) is orally administered on-demand in the prodromal phase of an acute HAE attack.

7. The compound of Formula A (or a pharmaceutically acceptable salt and/or solvate thereof) for use according to claim 6,
wherein the symptom recognised is at least one of: a slight swelling, abdominal pain or reddening of the skin.

8. The compound of Formula A (or a pharmaceutically acceptable salt and/or solvate thereof) for use according to claim 7,
wherein the symptom recognised is erythema marginatum.

9. The compound of Formula A (or a pharmaceutically acceptable salt and/or solvate thereof) for use according to any of claims 1 to 8,
wherein the treatment shortens the duration of the acute HAE attack.

10. The compound of Formula A (or a pharmaceutically acceptable salt and/or solvate thereof) for use according to claim 6,
wherein the treatment prevents the acute HAE attack from progressing to the swelling stage of an acute HAE attack.

11. The compound of Formula A (or a pharmaceutically acceptable salt and/or solvate thereof) for use according to claim 1,
wherein the compound of Formula A (or a pharmaceutically acceptable salt and/or solvate thereof) is orally administered on-demand to prophylactically reduce the likelihood of an acute HAE attack.

12. The compound of Formula A (or a pharmaceutically acceptable salt and/or solvate thereof) for use according to claim 11,
wherein the compound of Formula A (or a pharmaceutically acceptable salt and/or solvate thereof) is orally administered on-demand when it is anticipated that an acute HAE attack will be induced.

13. The compound of Formula A (or a pharmaceutically acceptable salt and/or solvate thereof) for use according to claim 12,
wherein the compound of Formula A (or a pharmaceutically acceptable salt and/or solvate thereof) is orally administered on-demand when it is anticipated that an acute HAE attack will be induced by physical traumata and/or stress.

14. The compound of Formula A (or a pharmaceutically acceptable salt and/or solvate thereof) for use according to claim 13,
wherein it is anticipated that an acute HAE attack will be induced by the physical traumata of a dental procedure and/or the mental stress associated with a dental procedure.

15. The compound of Formula A (or a pharmaceutically acceptable salt and/or solvate thereof) for use according to any of claims 11 to 14,
wherein the compound of Formula A (or a pharmaceutically acceptable salt and/or solvate thereof) is orally administered on-demand to prevent an acute HAE attack.

16. The compound of Formula A (or a pharmaceutically acceptable salt and/or solvate thereof) for use according to any preceding claim,
wherein the compound (or a pharmaceutically acceptable salt and/or solvate thereof) is administered as an oral dosage form comprising: (i) the compound (or a pharmaceutically acceptable salt and/or solvate thereof), and (ii) pharmaceutically acceptable excipients.

17. The compound of Formula A (or a pharmaceutically acceptable salt and/or solvate thereof) for use according to claim 16,
wherein the oral dosage form is a tablet comprising microcrystalline cellulose as a diluent, croscarmellose sodium as a disintegrant, polyvinyl pyrrolidone as a binder, and optionally magnesium stearate as a lubricant.

18. The compound of Formula A (or a pharmaceutically acceptable salt and/or solvate thereof) for use according to any preceding claim,
wherein the compound (or a pharmaceutically acceptable salt and/or solvate thereof) (i) inhibits plasma kallikrein, (ii) reduces cleavage of plasma prekallikrein, and/or (iii) reduces the generation of Factor XIIa from Factor XII.

19. The compound of Formula A (or a pharmaceutically acceptable salt and/or solvate thereof) for use according to claim 18,
wherein the patient is administered a dose of the compound of Formula A (or a pharmaceutically acceptable salt and/or solvate thereof) such that the patient's plasma has a concentration of the compound of Formula A (or a pharmaceutically acceptable salt and/or solvate thereof) of at least 500 ng/mL.

20. The compound of Formula A (or a pharmaceutically acceptable salt and/or solvate thereof) for use according to claim 19,
wherein the patient is administered at least 60 mg of the compound of Formula A (or a pharmaceutically acceptable salt and/or solvate thereof).

21. The compound of Formula A (or a pharmaceutically acceptable salt and/or solvate thereof) for use according to any preceding claim,
wherein the compound of Formula A (or a pharmaceutically acceptable salt and/or solvate thereof) blocks contact system activation for up to six hours.

22. The compound of Formula A (or a pharmaceutically acceptable salt and/or solvate thereof) for use according to any preceding claim,
wherein the compound of Formula A (or a pharmaceutically acceptable salt and/or solvate thereof) is administered at a daily dosage amount of between 5 mg and 2000 mg.

23. The compound of Formula A (or a pharmaceutically acceptable salt and/or solvate thereof) for use according to any preceding claim,
wherein the compound of Formula A is administered at a daily dosage amount of between 100 mg and 1500 mg, between 300 mg to 1800 mg, between 100 mg and 1400 mg per day, between 200 mg and 1200 mg, between 300 mg and 1200 mg, between 600 mg and 1200 mg, between 450 mg and 900 mg, between 500 mg and 1000 mg, between 450 mg and 600 mg, between 500 mg and 700 mg, between 800 mg and 1000 mg per day, between 900 mg and 1400 mg, or between 900 mg and 1200 mg.

24. The compound of Formula A (or a pharmaceutically acceptable salt and/or solvate thereof) for use according to any preceding claim,
wherein the patient is administered the daily dosage amount in two dosage amounts within a 24 hour period starting from the time of taking the first dosage amount.

25. The compound of Formula A (or a pharmaceutically acceptable salt and/or solvate thereof) for use according to claim 24,
wherein the two dosage amounts are administered simultaneously, separately or sequentially.

26. The compound of Formula A (or a pharmaceutically acceptable salt and/or solvate thereof) for use according to any of claims 24 or 25,
wherein the second dosage amount is administered between 2 and 6 hours of the first, preferably between about 3 and 6 hours of the first dosage amount.

27. The compound of Formula A (or a pharmaceutically acceptable salt and/or solvate thereof) for use according to any of claims 24 or 25,
wherein the second dosage amount can be administered at least about 6 hours after the first dosage amount.

28. The compound of Formula A (or a pharmaceutically acceptable salt and/or solvate thereof) for use according to any of claims 1 to 23,
wherein the patient is administered the compound of Formula A (or a pharmaceutically acceptable salt and/or solvate thereof) as three dosage amounts per day.

29. The compound of Formula A (or a pharmaceutically acceptable salt and/or solvate thereof) for use according to claim 28,
wherein the three dosage amounts are administered simultaneously, separately or sequentially.

30. The compound of Formula A (or a pharmaceutically acceptable salt and/or solvate thereof) for use according to any of claims 28 or 29,
wherein the second and third dosage amounts can be administered at least about 6 hours after the preceding dosage amount.

31. The compound of Formula A (or a pharmaceutically acceptable salt and/or solvate thereof) for use according to any of claims 21 to 30,
wherein each dosage amount comprises about 600 mg of the compound of formula A.

32. The compound of Formula A (or a pharmaceutically acceptable salt and/or solvate thereof) for use according to claim 31,
wherein each dosage amount is administered as two tablets each comprising about 300 mg of the compound of formula A.

## Patentansprüche

1. Verbindung der Formel A (oder ein pharmazeutisch akzeptables Salz und/oder Solvat davon) zur Verwendung bei der Bedarfsbehandlung von hereditärem Angioödem (HAE), umfassend: bedarfsweise orale Verabreichung der Verbindung der Formel A (oder eines pharmazeutisch akzeptablen Salzes und/oder Solvats davon) an einen Patienten, der diese benötigt,

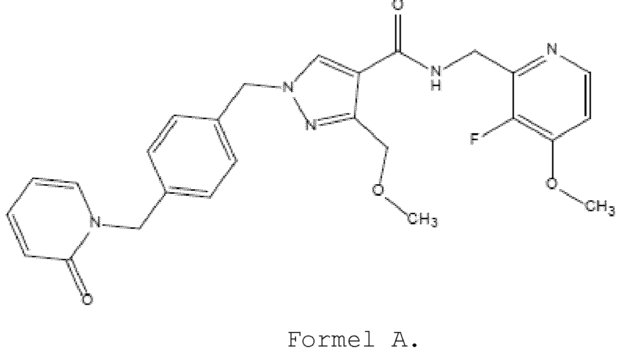

2. Verbindung der Formel A (oder ein pharmazeutisch akzeptables Salz und/oder Solvat davon) zur Verwendung nach Anspruch 1,
wobei die Verbindung der Formel A (oder ein pharmazeutisch akzeptables Salz und/oder Solvat davon) zur Verwendung bei der Bedarfsbehandlung einer akuten Attacke von hereditärem Angioödem (HAE) bestimmt ist und bei Erkennung eines Symptoms einer akuten HAE-Attacke als orale Bedarfsbehandlung verabreicht wird.

3. Verbindung der Formel A (oder ein pharmazeutisch akzeptables Salz und/oder Solvat davon) zur Verwendung nach Anspruch 2,
wobei das erkannte Symptom einer akuten HAE-Attacke mindestens eines der folgenden ist: Schwellung von Geweben; Müdigkeit; Kopfschmerzen; Muskelschmerzen; Kribbeln der Haut; Bauchschmerzen; Übelkeit; Erbrechen; Diarrhoe; Schluckstörungen; Heiserkeit; Kurzatmigkeit; und/oder Stimmungsschwankungen.

4. Verbindung der Formel A (oder ein pharmazeutisch akzeptables Salz und/oder Solvat davon) zur Verwendung nach einem der Ansprüche 2 oder 3,
wobei die Verbindung der Formel A (oder ein pharmazeutisch akzeptables Salz und/oder Solvat davon) innerhalb von 1 Stunde nach Erkennung des Symptoms einer akuten HAE-Attacke als orale Bedarfsbehandlung verabreicht wird.

5. Verbindung der Formel A (oder ein pharmazeutisch akzeptables Salz und/oder Solvat davon) zur Verwendung nach einem der Ansprüche 2 bis 4,
wobei die Verbindung der Formel A (oder ein pharmazeutisch akzeptables Salz und/oder Solvat davon) innerhalb von 30 Minuten, innerhalb von 20 Minuten, innerhalb von 10 Minuten oder innerhalb von 5 Minuten nach Erkennung des Symptoms einer akuten HAE-Attacke als orale Bedarfsbehandlung verabreicht wird.

6. Verbindung der Formel A (oder ein pharmazeutisch akzeptables Salz und/oder Solvat davon) zur Verwendung nach einem der Ansprüche 2 bis 5,
wobei die Verbindung der Formel A (oder ein pharmazeutisch akzeptables Salz und/oder Solvat davon) in der Prodromalphase einer akuten HAE-Attacke als orale Bedarfsbehandlung verabreicht wird.

7. Verbindung der Formel A (oder ein pharmazeutisch akzeptables Salz und/oder Solvat davon) zur Verwendung nach Anspruch 6,
wobei das erkannte Symptom mindestens eines der folgenden ist: leichte Schwellung, Bauchschmerzen oder Hautrötung.

8. Verbindung der Formel A (oder ein pharmazeutisch akzeptables Salz und/oder Solvat davon) zur Verwendung nach Anspruch 7,
wobei das erkannte Symptom Erythema marginatum ist.

9. Verbindung der Formel A (oder ein pharmazeutisch akzeptables Salz und/oder Solvat davon) zur Verwendung nach einem der Ansprüche 1 bis 8,
wobei die Behandlung die Dauer der akuten HAE-Attacke verkürzt.

10. Verbindung der Formel A (oder ein pharmazeutisch akzeptables Salz und/oder Solvat davon) zur Verwendung nach Anspruch 6,
wobei die Behandlung verhindert, dass die akute HAE-Attacke zum Schwellungsstadium einer akuten HAE-Attacke fortschreitet.

11. Verbindung der Formel A (oder ein pharmazeutisch akzeptables Salz und/oder Solvat davon) zur Verwendung nach Anspruch 1,
wobei die Verbindung der Formel A (oder ein pharmazeutisch akzeptables Salz und/oder Solvat davon) zur prophylaktischen Senkung der Wahrscheinlichkeit einer akuten HAE-Attacke als orale Bedarfsbehandlung verabreicht wird.

12. Verbindung der Formel A (oder ein pharmazeutisch akzeptables Salz und/oder Solvat davon) zur Verwendung nach Anspruch 11,
wobei die Verbindung der Formel A (oder ein pharmazeutisch akzeptables Salz und/oder Solvat davon) als orale Bedarfsbehandlung verabreicht wird, wenn vorhergesehen wird, dass eine akute HAE-Attacke ausgelöst wird.

13. Verbindung der Formel A (oder ein pharmazeutisch akzeptables Salz und/oder Solvat davon) zur Verwendung nach Anspruch 12,
wobei die Verbindung der Formel A (oder ein pharmazeutisch akzeptables Salz und/oder Solvat davon) als orale Bedarfsbehandlung verabreicht wird, wenn vorhergesehen wird, dass eine akute HAE-Attacke durch körperliches Trauma und/oder Stress ausgelöst wird.

14. Verbindung der Formel A (oder ein pharmazeutisch akzeptables Salz und/oder Solvat davon) zur Verwendung nach Anspruch 13,
wobei vorhergesehen wird, dass eine akute HAE-Attacke durch das körperliche Trauma eines zahnärztlichen Eingriffs und/oder durch den mit einem zahnärztlichen Eingriff assoziierten mentalen Stress ausgelöst wird.

15. Verbindung der Formel A (oder ein pharmazeutisch akzeptables Salz und/oder Solvat davon) zur Verwendung nach einem der Ansprüche 11 bis 14,
wobei die Verbindung der Formel A (oder ein pharmazeutisch akzeptables Salz und/oder Solvat davon) zur Verhinderung einer akuten HAE-Attacke als orale Bedarfsbehandlung verabreicht wird.

16. Verbindung der Formel A (oder ein pharmazeutisch akzeptables Salz und/oder Solvat davon) zur Verwendung nach einem der vorhergehenden Ansprüche,
wobei die Verbindung (oder ein pharmazeutisch akzeptables Salz und/oder Solvat davon) als orale Darreichungsform verabreicht wird, umfassend: (i) die Verbindung (oder das pharmazeutisch akzeptable Salz und/oder Solvat davon) und (ii) pharmazeutisch akzeptable Hilfsstoffe.

17. Verbindung der Formel A (oder ein pharmazeutisch akzeptables Salz und/oder Solvat davon) zur Verwendung nach Anspruch 16,
wobei die orale Darreichungsform eine Tablette ist, die mikrokristalline Cellulose als ein Verdünnungsmittel, Croscarmellose-Natrium als ein Sprengmittel, Polyvinylpyrrolidon als ein Bindemittel und optional Magnesiumstearat als ein Gleitmittel umfasst.

18. Verbindung der Formel A (oder ein pharmazeutisch akzeptables Salz und/oder Solvat davon) zur Verwendung nach einem der vorhergehenden Ansprüche,
wobei die Verbindung (oder ein pharmazeutisch akzeptables Salz und/oder Solvat davon) (i) Plasmakallikrein unterdrückt, (ii) Spaltung von Prekallikrein in Plasma verringert und/oder (iii) die Erzeugung von Faktor XIIA aus Faktor XII verringert.

19. Verbindung der Formel A (oder ein pharmazeutisch akzeptables Salz und/oder Solvat davon) zur Verwendung nach Anspruch 18,
wobei dem Patienten eine Dosis der Verbindung der Formel A (oder eines pharmazeutisch akzeptablen Salzes und/oder Solvats davon) verabreicht wird, so dass das Plasma des Patienten eine Konzentration der Verbindung der Formel A (oder eines pharmazeutisch akzeptablen Salzes und/oder Solvats davon) von mindestens 500 ng/ml aufweist.

20. Verbindung der Formel A (oder ein pharmazeutisch akzeptables Salz und/oder Solvat davon) zur Verwendung nach Anspruch 19,
wobei dem Patienten mindestens 60 mg der Verbindung der Formel A (oder eines pharmazeutisch akzeptablen Salzes und/oder Solvats davon) verabreicht werden.

21. Verbindung der Formel A (oder ein pharmazeutisch akzeptables Salz und/oder Solvat davon) zur Verwendung nach einem der vorhergehenden Ansprüche,
wobei die Verbindung der Formel A (oder ein pharmazeutisch akzeptables Salz und/oder Solvat davon) die Kontaktsystemaktivierung für bis zu sechs Stunden blockiert.

22. Verbindung der Formel A (oder ein pharmazeutisch akzeptables Salz und/oder Solvat davon) zur Verwendung nach einem der vorhergehenden Ansprüche,
wobei die Verbindung der Formel A (oder ein pharmazeutisch akzeptables Salz und/oder Solvat davon) in einer Tagesdosismenge zwischen 5 mg und 2000 mg verabreicht wird.

23. Verbindung der Formel A (oder ein pharmazeutisch akzeptables Salz und/oder Solvat davon) zur Verwendung nach einem der vorhergehenden Ansprüche,
wobei die Verbindung der Formel A in einer Tagesdosismenge zwischen 100 mg und 1500 mg, zwischen 300 mg bis 1800 mg, zwischen 100 mg und 1400 mg pro Tag, zwischen 200 mg und 1200 mg, zwischen 300 mg und 1200 mg, zwischen 600 mg und 1200 mg, zwischen 450 mg und 900 mg, zwischen 500 mg und 1000 mg, zwischen 450 mg und 600 mg, zwischen 500 mg und 700 mg, zwischen 800 mg und 1000 mg pro Tag, zwischen 900 mg und 1400 mg, oder zwischen 900 mg und 1200 mg verabreicht wird.

24. Verbindung der Formel A (oder ein pharmazeutisch akzeptables Salz und/oder Solvat davon) zur Verwendung nach einem der vorhergehenden Ansprüche,
wobei dem Patienten die Tagesdosismenge in zwei Dosismengen innerhalb eines 24-Stunden-Zeitraums ab dem Zeitpunkt der Einnahme der ersten Dosismenge verabreicht wird.

25. Verbindung der Formel A (oder ein pharmazeutisch akzeptables Salz und/oder Solvat davon) zur Verwendung nach Anspruch 24,
wobei die zwei Dosismengen gleichzeitig, getrennt oder nacheinander verabreicht werden.

26. Verbindung der Formel A (oder ein pharmazeutisch akzeptables Salz und/oder Solvat davon) zur Verwendung nach einem der Ansprüche 24 oder 25,
wobei die zweite Dosismenge zwischen 2 und 6 Stunden nach der ersten, vorzugsweise zwischen ungefähr 3 und 6 Stunden nach der ersten Dosismenge verabreicht wird.

27. Verbindung der Formel A (oder ein pharmazeutisch akzeptables Salz und/oder Solvat davon) zur Verwendung nach einem der Ansprüche 24 oder 25,
wobei die zweite Dosismenge mindestens ungefähr 6 Stunden nach der ersten Dosismenge verabreicht werden kann.

28. Verbindung der Formel A (oder ein pharmazeutisch akzeptables Salz und/oder Solvat davon) zur Verwendung nach einem der Ansprüche 1 bis 23,
wobei dem Patienten die Verbindung der Formel A (oder ein pharmazeutisch akzeptables Salz und/oder Solvat davon) als drei Dosismengen pro Tag verabreicht wird.

29. Verbindung der Formel A (oder ein pharmazeutisch akzeptables Salz und/oder Solvat davon) zur Verwendung nach Anspruch 28,
wobei die drei Dosismengen gleichzeitig, getrennt oder nacheinander verabreicht werden.

30. Verbindung der Formel A (oder ein pharmazeutisch akzeptables Salz und/oder Solvat davon) zur Verwendung nach einem der Ansprüche 28 oder 29,
wobei die zweiten und dritten Dosismengen mindestens ungefähr 6 Stunden nach der vorausgehenden Dosismenge verabreicht werden können.

31. Verbindung der Formel A (oder ein pharmazeutisch akzeptables Salz und/oder Solvat davon) zur Verwendung nach einem der Ansprüche 21 bis 30,
wobei jede Dosismenge ungefähr 600 mg der Verbindung der Formel A umfasst.

32. Verbindung der Formel A (oder ein pharmazeutisch akzeptables Salz und/oder Solvat davon) zur Verwendung nach Anspruch 31,
wobei jede Dosismenge als zwei Tabletten verabreicht wird, die jeweils ungefähr 300 mg der Verbindung der Formel A umfassen.

## Revendications

1. Composé de formule A (ou un sel pharmaceutiquement acceptable et/ou solvate de celui-ci) destiné à être utilisé en traitement d'angioœdème héréditaire (AOH) à la demande comprenant : l'administration par voie orale du composé de formule A (ou d'un sel pharmaceutiquement acceptable et/ou solvate de celui-ci) à un patient qui en a besoin à la demande,

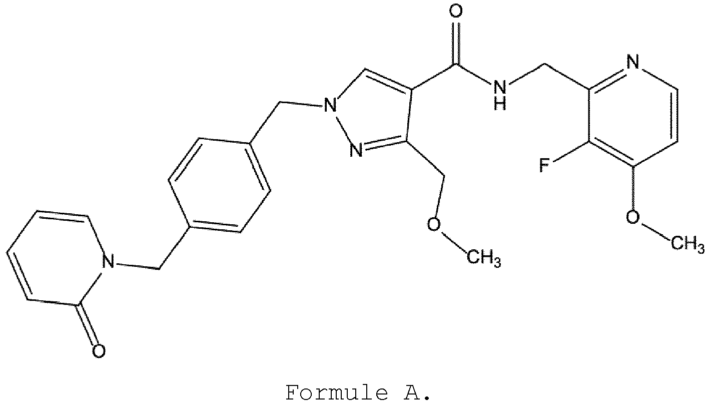

2. Composé de formule A (ou un sel pharmaceutiquement acceptable et/ou solvate de celui-ci) destiné à être utilisé selon la revendication 1,
le composé de formule A (ou un sel pharmaceutiquement acceptable et/ou solvate de celui-ci) étant destiné à être utilisé en traitement d'une crise aiguë d'angioœdème héréditaire (AOH) à la demande et étant administré par voie orale à la demande lors de la reconnaissance d'un symptôme d'une crise d'AOH aiguë.

3. Composé de formule A (ou un sel pharmaceutiquement acceptable et/ou solvate de celui-ci) destiné à être utilisé selon la revendication 2,
le symptôme d'une crise d'AOH aiguë reconnu étant au moins l'un de : un gonflement de tissus ; une fatigue ; un mal de tête ; des douleurs musculaires ; un picotement sur la peau ; une douleur abdominale ; la nausée ; des vomissements ; la diarrhée ; une difficulté à avaler ; un enrouement ; un essoufflement ; et/ou des changements d'humeur.

4. Composé de formule A (ou un sel pharmaceutiquement acceptable et/ou solvate de celui-ci) destiné à être utilisé selon l'une quelconque des revendications 2 ou 3,
le composé de formule A (ou un sel pharmaceutiquement acceptable et/ou solvate de celui-ci) étant administré par voie orale à la demande moins de 1 heure après que le symptôme d'une crise d'AOH aiguë a été reconnu.

5. Composé de formule A (ou un sel pharmaceutiquement acceptable et/ou solvate de celui-ci) destiné à être utilisé selon l'une quelconque des revendications 2 à 4,
le composé de formule A (ou un sel pharmaceutiquement acceptable et/ou solvate de celui-ci) étant administré par voie orale à la demande moins de 30 minutes, moins de 20 minutes, moins de 10 minutes ou moins de 5 minutes après que le symptôme d'une crise d'AOH aiguë a été reconnu.

6. Composé de formule A (ou un sel pharmaceutiquement acceptable et/ou solvate de celui-ci) destiné à être utilisé selon l'une quelconque des revendications 2 à 5,
le composé de formule A (ou un sel pharmaceutiquement acceptable et/ou solvate de celui-ci) étant administré par voie orale à la demande dans la phase prodromique d'une crise d'AOH aiguë.

7. Composé de formule A (ou un sel pharmaceutiquement acceptable et/ou solvate de celui-ci) destiné à être utilisé selon la revendication 6,
le symptôme reconnu étant au moins l'un de : un léger gonflement, une douleur abdominale ou un rougissement de la peau.

8. Composé de formule A (ou un sel pharmaceutiquement acceptable et/ou solvate de celui-ci) destiné à être utilisé selon la revendication 7,
le symptôme reconnu étant un érythème marginé.

9. Composé de formule A (ou un sel pharmaceutiquement acceptable et/ou solvate de celui-ci) destiné à être utilisé selon l'une quelconque des revendications 1 à 8,
le traitement raccourcissant la durée de la crise d'AOH aiguë.

10. Composé de formule A (ou un sel pharmaceutiquement acceptable et/ou solvate de celui-ci) destiné à être utilisé selon la revendication 6,
le traitement empêchant la crise d'AOH aiguë de progresser au stade de gonflement d'une crise d'AOH aiguë.

11. Composé de formule A (ou un sel pharmaceutiquement acceptable et/ou solvate de celui-ci) destiné à être utilisé selon la revendication 1,
le composé de formule A (ou un sel pharmaceutiquement acceptable et/ou solvate de celui-ci) étant administré par voie orale à la demande pour réduire de manière prophylactique la probabilité d'une crise d'AOH aiguë.

12. Composé de formule A (ou un sel pharmaceutiquement acceptable et/ou solvate de celui-ci) destiné à être utilisé selon la revendication 11,
le composé de formule A (ou un sel pharmaceutiquement acceptable et/ou solvate de celui-ci) étant administré par voie orale à la demande lorsqu'on s'attend à ce qu'une crise d'AOH aiguë soit déclenchée.

13. Composé de formule A (ou un sel pharmaceutiquement acceptable et/ou solvate de celui-ci) destiné à être utilisé selon la revendication 12,
le composé de formule A (ou un sel pharmaceutiquement acceptable et/ou solvate de celui-ci) étant administré par voie orale à la demande lorsqu'on s'attend à ce qu'une crise d'AOH aiguë soit déclenchée par un traumatisme physique et/ou du stress.

14. Composé de formule A (ou un sel pharmaceutiquement acceptable et/ou solvate de celui-ci) destiné à être utilisé selon la revendication 13,
sachant qu'on s'attend à ce qu'une crise d'AOH aiguë soit déclenchée par le traumatisme physique d'un acte dentaire et/ou le stress mental associé à un acte dentaire.

15. Composé de formule A (ou un sel pharmaceutiquement acceptable et/ou solvate de celui-ci) destiné à être utilisé selon l'une quelconque des revendications 11 à 14,
le composé de formule A (ou un sel pharmaceutiquement acceptable et/ou solvate de celui-ci) étant administré par voie orale à la demande pour empêcher une crise d'AOH aiguë.

16. Composé de formule A (ou un sel pharmaceutiquement acceptable et/ou solvate de celui-ci) destiné à être utilisé selon une quelconque revendication précédente,
le composé (ou un sel pharmaceutiquement acceptable et/ou solvate de celui-ci) étant administré sous une forme galénique orale comprenant : (i) le composé (ou un sel pharmaceutiquement acceptable et/ou solvate de celui-ci) et (ii) des excipients pharmaceutiquement acceptables.

17. Composé de formule A (ou un sel pharmaceutiquement acceptable et/ou solvate de celui-ci) destiné à être utilisé selon la revendication 16,
la forme galénique orale étant un comprimé comprenant de la cellulose microcristalline en tant que diluant, de la croscarmellose sodique en tant que délitant, de la polyvinylpyrrolidone en tant que liant et éventuellement du stéarate de magnésium en tant que lubrifiant.

18. Composé de formule A (ou un sel pharmaceutiquement acceptable et/ou solvate de celui-ci) destiné à être utilisé selon une quelconque revendication précédente,
le composé (ou un sel pharmaceutiquement acceptable et/ou solvate de celui-ci) (i) inhibant la kallicréine plasmatique, (ii) réduisant le clivage de kallicréine plasmatique et/ou (iii) réduisant la formation de facteur XIIa à partir de facteur XII.

19. Composé de formule A (ou un sel pharmaceutiquement acceptable et/ou solvate de celui-ci) destiné à être utilisé selon la revendication 18,
une dose du composé de formule A (ou d'un sel pharmaceutiquement acceptable et/ou solvate de celui-ci) étant administrée au patient de telle sorte que le plasma du patient a une concentration du composé de formule A (ou d'un sel pharmaceutiquement acceptable et/ou solvate de celui-ci) d'au moins 500 ng/ml.

20. Composé de formule A (ou un sel pharmaceutiquement acceptable et/ou solvate de celui-ci) destiné à être utilisé selon la revendication 19,
au moins 60 mg du composé de formule A (ou d'un sel pharmaceutiquement acceptable et/ou solvate de celui-ci) étant administré au patient.

21. Composé de formule A (ou un sel pharmaceutiquement acceptable et/ou solvate de celui-ci) destiné à être utilisé selon une quelconque revendication précédente,
le composé de formule A (ou un sel pharmaceutiquement acceptable et/ou solvate de celui-ci) bloquant l'activation du système de contact pendant jusqu'à six heures.

22. Composé de formule A (ou un sel pharmaceutiquement acceptable et/ou solvate de celui-ci) destiné à être utilisé selon une quelconque revendication précédente,
le composé de formule A (ou un sel pharmaceutiquement acceptable et/ou solvate de celui-ci) étant administré en une quantité posologique journalière comprise entre 5 mg et 2000 mg.

23. Composé de formule A (ou un sel pharmaceutiquement acceptable et/ou solvate de celui-ci) destiné à être utilisé selon une quelconque revendication précédente,
le composé de formule A étant administré en une quantité posologique journalière comprise entre 100 mg et 1500 mg, entre 300 mg et 1800 mg, entre 100 mg et 1400 mg par jour, entre 200 mg et 1200 mg, entre 300 mg et 1200 mg, entre 600 mg et 1200 mg, entre 450 mg et 900 mg, entre 500 mg et 1000 mg, entre 450 mg et 600 mg, entre 500 mg et 700 mg, entre 800 mg et 1000 mg par jour, entre 900 mg et 1400 mg ou entre 900 mg et 1200 mg.

24. Composé de formule A (ou un sel pharmaceutiquement acceptable et/ou solvate de celui-ci) destiné à être utilisé selon une quelconque revendication précédente,
la quantité posologique journalière étant administrée au patient en deux quantités posologiques sur une période de moins de 24 heures à partir du moment de la prise de la première quantité posologique.

25. Composé de formule A (ou un sel pharmaceutiquement acceptable et/ou solvate de celui-ci) destiné à être utilisé selon la revendication 24,
les deux quantités posologiques étant administrées simultanément, séparément ou séquentiellement.

26. Composé de formule A (ou un sel pharmaceutiquement acceptable et/ou solvate de celui-ci) destiné à être utilisé selon l'une quelconque des revendications 24 ou 25,
la seconde quantité posologique étant administrée à entre 2 et 6 heures de la première, de préférence entre environ 3 et 6 heures de la première quantité posologique.

27. Composé de formule A (ou un sel pharmaceutiquement acceptable et/ou solvate de celui-ci) destiné à être utilisé selon l'une quelconque des revendications 24 ou 25,
la seconde quantité posologique pouvant être administrée au moins environ 6 heures après la première quantité posologique.

28. Composé de formule A (ou un sel pharmaceutiquement acceptable et/ou solvate de celui-ci) destiné à être utilisé selon l'une quelconque des revendications 1 à 23,
le composé de formule A (ou un sel pharmaceutiquement acceptable et/ou solvate de celui-ci) étant administré au patient sous forme de trois quantités posologiques par jour.

29. Composé de formule A (ou un sel pharmaceutiquement acceptable et/ou solvate de celui-ci) destiné à être utilisé selon la revendication 28,
les trois quantités posologiques étant administrées simultanément, séparément ou séquentiellement.

30. Composé de formule A (ou un sel pharmaceutiquement acceptable et/ou solvate de celui-ci) destiné à être utilisé selon l'une quelconque des revendications 28 ou 29,
les deuxième et troisième quantités posologiques pouvant être administrées au moins environ 6 heures après la quantité posologique précédente.

31. Composé de formule A (ou un sel pharmaceutiquement acceptable et/ou solvate de celui-ci) destiné à être utilisé selon l'une quelconque des revendications 21 à 30,
chaque quantité posologique comprenant environ 600 mg du composé de formule A.

32. Composé de formule A (ou un sel pharmaceutiquement acceptable et/ou solvate de celui-ci) destiné à être utilisé selon la revendication 31,
chaque quantité posologique étant administrée sous forme de deux comprimés comprenant chacun environ 300 mg du composé de formule A.
